# EUROPEAN PATENT SPECIFICATION

(11) **EP 1 554 304 B1**
(45) Date of publication and mention of the grant of the patent: **10.06.2009**
(21) Application number: 03714589.3
(22) Date of filing: 23.04.2003
(51) Int. Cl.: C07J 63/00, A61P 35/00, A61P 35/02, A61K 31/565

(54) **SHIP 1 MODULATORS**
MODULATOREN VON SHIP-1
MODULATEURS DE SHIP 1

(30) Priority: 17.10.2002 WO PCT/CA02/01550
(43) Date of publication of application: 20.07.2005
(73) Proprietor: THE UNIVERSITY OF BRITISH COLUMBIA, Vancouver, British Columbia V6T 1Z3 (CA)
(72) Inventor: ANDERSEN, Raymond, Vancouver, British Columbia V6S 1Z6 (CA); WILLIAMS, David, E., Vancouver, British Columbia V5T 2T7 (CA); MUI, Alice, Burnaby, British Columbia V5E 2E7 (CA); ONG, Christopher, Vancouver, British Columbia V6Z 2X4 (CA); KRYSTAL, Gerald, Vancouver, British Columbia V6N 1A3 (CA); YANG Lu, San Diego, California 92122 (US)
(74) Representative: Brasnett, Adrian Hugh
(86) International application number: PCT/CA2003/000571
(87) International publication number: WO 2004/035601

(56) References cited:
- WO-A-03/033517
- GOCLIK, EVA ET AL: "Pelorol from the Tropical Marine Sponge Dactylospongia elegans" JOURNAL OF NATURAL PRODUCTS (2000), 63(8), 1150-1152 , XP002254980 cited in the application
- HE W ET AL: "Novel cytokine release inhibitors. Part III: truncated analogs of tripterine" BIOORGANIC & MEDICINAL CHEMISTRY LETTERS, OXFORD, GB, vol. 8, no. 24, 15 December 1998 (1998-12-15), pages 3659-3664, XP004150387 ISSN: 0960-894X
- DATABASE CA [Online] CHEMICAL ABSTRACTS SERVICE, COLUMBUS, OHIO, US; SOROKINA, I. B. ET AL: "Estrogen and antineoplastic activity in a series of transformed estrone and estradiol analogs" retrieved from STN Database accession no. 80:91450 XP002230773 & IZVESTIYA AKADEMII NAUK SSSR, SERIYA BIOLOGICHESKAYA (1973), (5), 664-70,

## Description

### Technical Field of the Invention

The present invention relates to SHIP 1, a negative regulator of cell proliferation and survival and immune cell activation.

### Background of the Invention

SH₂-containing inositol 5-phosphatase (SHIP 1), selectively hydrolyzes the 5-phosphate from inositol 1,3,4,5-tetraphosphate (IP4) and phosphatalidylinositol 3,4,5-triphosphate (PIP3). United States Patent No. 6,238,903 discloses that SHIP 1 is an enzyme regulator of signaling pathways that control gene expression, cell proliferation, differentiation, activation, and metabolism, particularly of the Ras and phospholipid signaling pathways. SHIP 1 plays an important role in cytokine and immune receptor signal tansduction. SHIP 1 disrupted (SHIP 1 -/-) mice exhibit a myeloproliferative phenotype characterized by overproduction of granulocytes and macrophages¹. SHIP 1-/mast cells are more prone to IgE and Steel factor induced degranulation, while SHIP 1-/B cells are resistant to negative regulation by Fc RIIB. SHIP 1 is also involved in the pathogenesis of chronic myelogenous leukemia².

Compounds that specifically modulate the activity of SHIP 1 would be useful in the treatment of cell proliferation, hematopoietic and immune disorders, as well as for manipulating SHIP 1 mediated pathways during investigatory and drug discovery testing. To date, no structure of a small molecule SHIP 1 specific modulator has been disclosed.

A sesquiterpene compound termed pelorol may be obtained from various marine sponge species, including *Petrosaspongia metachromia* and *Dactylospongia elegans*. Kwak et al. and Goclik et al. each disclosed the structure of pelorol and its extraction from different marine sponges.^{4,5} Pelorol was reported as having weak antitrypanosomal and antiplasmodial effects⁵. The precise structure of pelorol is as follows, with Me representing a methyl group and relative configuration of chiral atoms (C-5, 8, 9 and 10) shown.

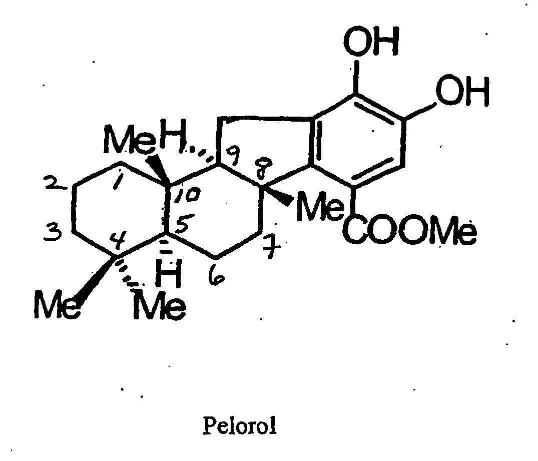

Some reduced and substituted chrysene derivatives similar to pelorol and having the characteristic *gem* substituted non-aromatic ring of pelorol are known as intermediates or derivatives in the preparation of various polycyclic polyprenols found in shale⁶⁻¹², in the preparation of taxodione¹³, and in the compound 1,2,3,4,4a,4b,5,6,10b,11,12,12a-dodecahydro-1,1-dimethyl-chrysene¹⁴. None of these chrysene derivatives are known to have biological activity.

### Summary of the Invention

This invention is based on the discovery that pelorol and related compounds are capable of modulation of SHIP 1 activity.

Some embodiments of this invention provide novel compounds of Formula I and salts thereof. Compounds of Formula I have the structure: 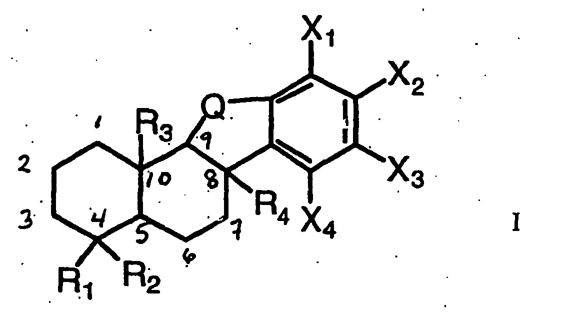 wherein;
R₁ and R₂ are independently selected from the group consisting of: -CH₃, -CH₂CH₃, -CH₂OH, -CH₂OR', -CHO, -CO₂H, and -CO₂R';
R₃ and R₄ are independently selected from the group consisting of: H, -CH₃, -CH₂CH₃, -CH₂OH, -CH₂OR', -CHO, -CO₂H, and -CO₂R';
Q is selected from the group consisting of: -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂-, and -CY₁Y₂CY₃Y₄CY₅Y₆-; where Y₁, Y₂, Y₃, Y₄, Y₅, and Y₆ are independently selected from the group consisting of: H, F, Br, Cl, I, OH, OR', and SH; or any one group of Y₁/Y₂, Y₃/Y₄, and Y₅/Y₆ may be =O; or Y₁/Y₃ may form an epoxide; and, at least one of Y₁, Y₂, Y₃, Y₄, Y₅ and Y₆ when present, is not H;
X₁, X₂, X₃, and X₄ are independently selected from the group consisting of: H, R, OH, -OR, -CO₂H, -CO₂R', F, Br, Cl, I, -CN, -SO₃H, -OSO₃H, NO₂, NH₂, -NHR, and -NR₂; where R is a linear, branched, or cyclic, saturated or unsaturated one to ten carbon alkyl group that is unsubstituted or is substituted with one or more of: OH, =O, SH, F, Br, Cl, I, NH₂, -NHR', -NR'₂, NO₂, -CO₂H, -CO₂R', and epoxide;
and R' is a linear, branched, or cyclic, saturated or unsaturated one to ten carbon alkyl group that is unsubstituted or substituted with one or more of: OH, =O, SH, F, Br, Cl, I, NH₂, -NHR", -NR"₂, NO₂ and -CO₂H where R" is a linear, branched, or cyclic, saturated or unsaturated one to ten carbon alkyl group.

Novel compounds of Formula I of this invention do not include the precise structures of previously described gem substituted chrysene derivatives. These previously described compounds include pelorol and compounds having the following structures in which Me is methyl:

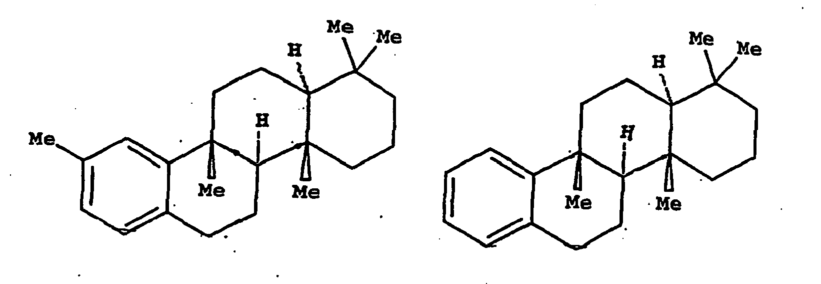

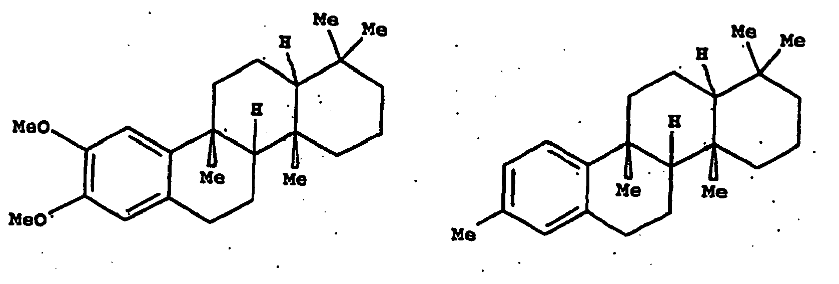

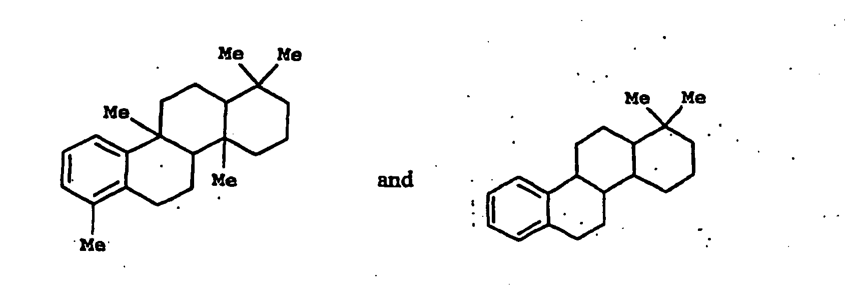

Alternately defined, this invention excludes such previously known specific compounds of Formula I in which each of R₁ - R₄ are methyl; Q is -CH₂CH₂; and, X₁ - X₄ is according to any one of the following definitions:
(a) X₁ and X₂ = OR, X₃ = H, and X₄ = -COOCH₃;
(b) X₁, X₂, X₃ and X₄ = H;
(c) X₁, X₂, and X₄ = H, and X₃ = CH₃;
(d) X₁, X₃, and X₄ = H, and X₂ = CH₃;
(e) X₂, X₃, and X₄ = H, and X₁ = CH₃; and
(f) X₁ and X₄ = H, X₂ and X₃ = OCH₃.
Also excluded is a compound of Formula I in which R₁ and R₂ = CH₃; R₃ and R₄ = H; Q = -CH₂CH₂-; and each of X₁ - X₄ is H.

Some embodiments of this invention provide a pharmaceutical composition comprising one or more compounds of Formula I or pharmaceutically acceptable salts thereof, and a pharmaceutically acceptable carrier. Such compositions may comprise previously known compounds of Formula I which have not been known as biologically active compounds suitable for pharmaceutical use.

Some embodiments of this invention provide a compound of the invention, or a composition comprising a compound of formula I for use in a method of treatment or prevention of an immune, inflammatory, or neoplastic disorder or condition.

Some embodiments of this invention provide the use of a compound of Formula I or pharmaceutically acceptable salt thereof for modulation of SHIP 1 activity and for preparation of agents for the modulation of SHIP 1 activity. Such modulation may be *in vitro* or *in vivo.* Agents for *in vivo* use include a pharmaceutical composition of this invention as well as agents adapted for *in vitro* use. The modulation may be for a treatment or prevention of an immune, inflammatory, or neoplastic condition or disorders as described above.

Some compounds of Formula I may be prepared in whole or in part by fractionating biological extracts or by derivatizing available compounds. Alternately, compounds of Formula I may be prepared by total synthesis.

Some embodiments of this invention provide a method of making a compound of Formula IA 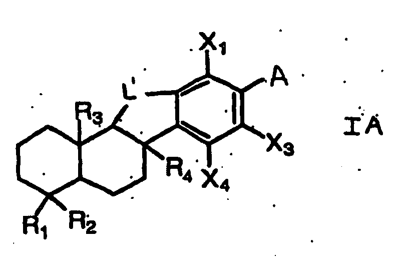 in which R₁ - R₄, X₁, X₃, and X₄ are as defined for Formula I, L' is a C₁-C₃ saturated or unsaturated alkyl linking group; and A is an activating group; comprising reacting a compound of Formula IIB: 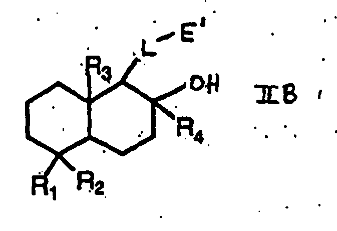 in which L is absent or is a C₁ - C₃ saturated or unsaturated alkyl linking group and E' is an electrophilic reactive group; with a compound of Formula III 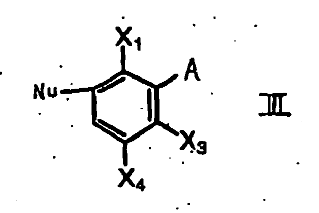 in which Nu is a group that renders the compound of Formula III nucleophilic at Nu, followed by optional reduction and by hydrolysis, to produce a compound of Formula IV 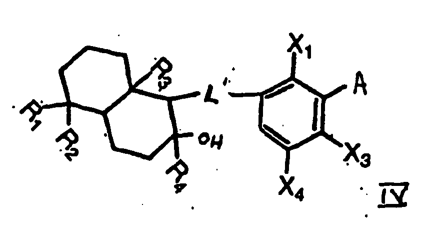 and condensing the compound of Formula IV to produce a compound of Formula IA.

L' in compounds of Formula IA may optionally be changed or derivatized to form a desired component Q of Formula I. For example, component L' in compounds of Formula IA produced by the preceding method may have different degrees of saturation or different substituents as compared to Q in a compound of Formula I. In order to reduce the number of atoms in the ring, a compound having an unsaturated L' group could be subjected to oxidizing and reduction steps to reduce the size of the ring in Formula I comprising Q. In addition, functionalities such as ketone, hydroxyl, or other groups may be added to L' to form a desired Q component.

Preferred electrophilic reactive groups for E are lactone, ester, and thioester. A preferred group for E' is carboxyl. More preferably, compounds of Formulas IIB are as follows.

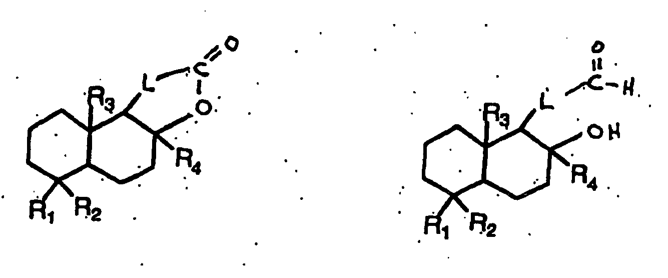

Even more preferably, compounds of Formulas IIB are as follows:

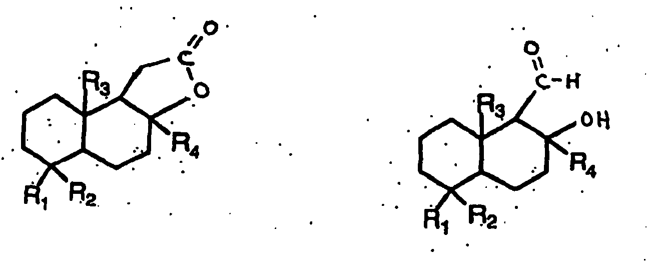

A preferred Nu in compounds of Formula III is lithium which may be substituted onto the ring for a halogen such as bromine. Preferably, A in the compound of Formula III is an activating group such as -OMe or NHAc (Me = methyl and Ac = acetyl) which group may be subsequently converted to a desired substituent for X₂ in compounds of Formula I. Substitutents may also be protected, where appropriate with a protecting group such as TBS.

### Brief Description of the Drawings

Figure 1 is a graph depicting the effect of sponge extracts on SHIP 1 enzyme activity *in vitro*.
Figure 2 is a graph depicting the effect of pelorol on macrophage nitric oxide (NO) production.
Figure 3 is a graph depicting the effect of pelorol on IgE mediated mast cell activation.

### Detailed Description of the Invention

In this specification, the following abbreviations will appear: THF (tetrahydrofuran); n-buLi (n-butyly lithium); t-buLi (tert-butyly lithium); Ph₃PMe (methyl triphenyl posphonium bromide); PCC (pyridinium chlorochromate); Ac (acetyl); Me (methyl); Et (ethyl); prop. (propyl); but. (butyl); RT or, r.t. (room temperature); hr. (hour(s)); DMSO (dimethylsulfoxide); DNFB (2,4-dinitrofluorobenzene); LPS (lipopolysaccarhide); TNF-α (Tumor Necrosis Factor Alpha); TBS (tert-butyl dimethylsilyl); and EA (ethyl acetate).

### SHIP 1 Modulating Compounds

Compounds of Formula I have chiral centres at C-5, C-8, C-9 and C-10 and may be chiral at C-4 depending upon whether R₁ and R₂ are different. Compounds of this invention include all stereoisomers and enantiomers of compounds of Formula I. Some embodiments have the same relative configuration of chiral centres as does pelorol or are enantiomers thereof, namely: S, R, R, S; or R, S, S, R (at C-5, 8, 9 and 10 respectively). Some embodiments have the same absolute configuration as pelorol at chiral centres. Some embodiments have the same relative configuration as pelorol at C-5 and C-10 with independently variable configurations at C-8 and C-9. Some embodiments have the same relative configuration as pelorol at C-5, C-8, and C-10 with variable configuration at C-9. In all cases, the configuration at C-4 (if chiral) may be variable or may be the same relative configuration to the remaining chiral centres as is shown in examples of structures of compounds of Formula I illustrated herein.

In various embodiments of this invention, the compounds may have the limitations in Formula I described above or may have more specific limitations with respect to substituents Q, R₁ - R₄, and X₁ - X₄. Any combination of the following limitations is encompassed by this invention.
(a) Q may be as defined for Formula I except that Y₁₋₆ is limited to H or halogen;
(b) Q may be limited to -CH₂-, -CH₂CH₂-, -CH=CH, -CH₂-CH₂CH₂- and -CH=CHCH₂-;
(c) Q may be limited to H or saturated moieties in the limitation of Formula I, or according to the limitations of paragraph (a) or (b) above;
(d) Q may be limited to a one or two carbon skeleton within the limitations of Formula I, or according to the limitations of any of paragraphs (a) - (c) above;
(e) one or both of R₁ and R₂ may be limited to methyl, ethyl, -CH₂OH or -CH₂OR';
(f) R' in one or both of R₁ and R₂ according to Formula I, or the limitation of paragraph (e) above, may be limited to methyl, ethyl, propyl or butyl;
(g) one or both of R₁ and R₂ may be limited to methyl or ethyl;
(h) one or both of R₁ and R₂ may be limited to methyl;
(i) R and R' in any one or more of X₁ - X₄ may be limited to unsubstituted methyl, ethyl, propyl or butyl;
(j) one or more of X₁ - X₃ may be limited to H, R, OH, OR, halogen, -CONH₂, -CONHR', -COR'₂, NHR or NR₂ where R and R' are limited as in Formula I, or R and R' may be according to paragraph (i) above;
(k) one or more of X₁ - X₃ is limited to H, OH, OR, -CONH₂, -CONHR', and -COR'₂, where R and R' are as in Formula I, or R and R' may be limited according to paragraph (i) above;
(l) one or more of X₁ - X₃ may be limited to H, OH, and OCH₃;
(m) X₄ may be limited to H, R, OH, OR, CO₂H or -CO₂R', with R and R' as in Formula I, or R and R' may be limited according to paragraph (i) above;
(n) X₄ may be limited to H, R, OH, OCH₃, -CO₂H and -CO₂R' with R and R' limited according to paragraph (i) above; and,
(o) X₄ may be limited to H, R, OH, OCH₃, -CO₂H or -CO₂CH₃.

The following specific structures are embodiments of this invention. In some cases, variability at X₁, X₂, and X₄ is shown with reference to substituents identified as R, Z, and Y, which for the purposes of the illustrated compounds are defined below. Although relative stereochemistry is illustrated for each structure, the configuration of chiral centres may vary according to any of the embodiments based on chirality described above.

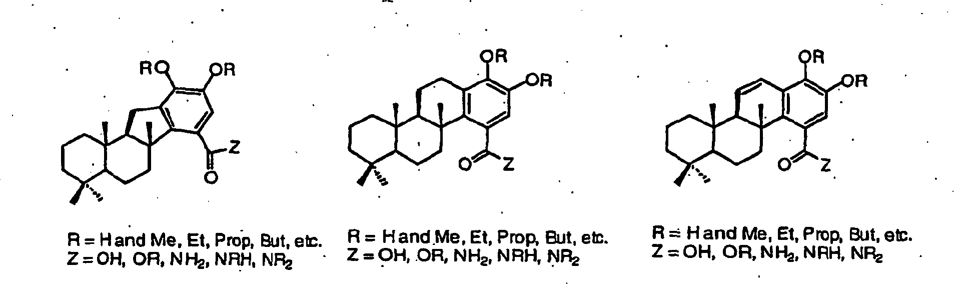

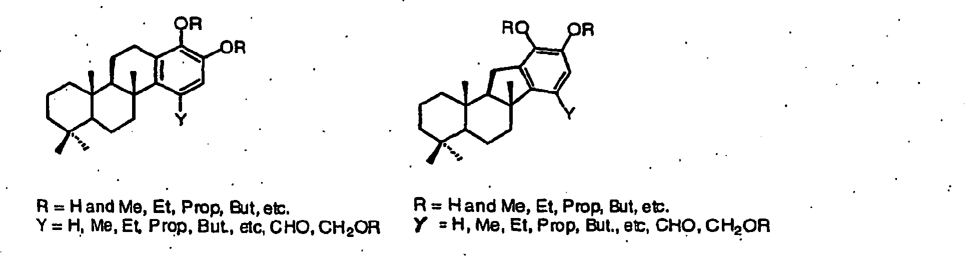

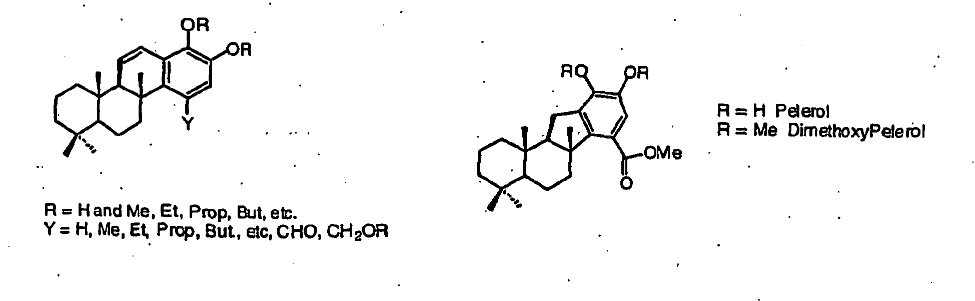

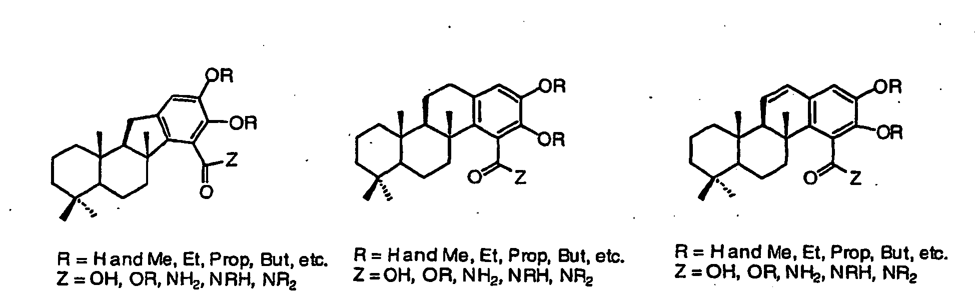

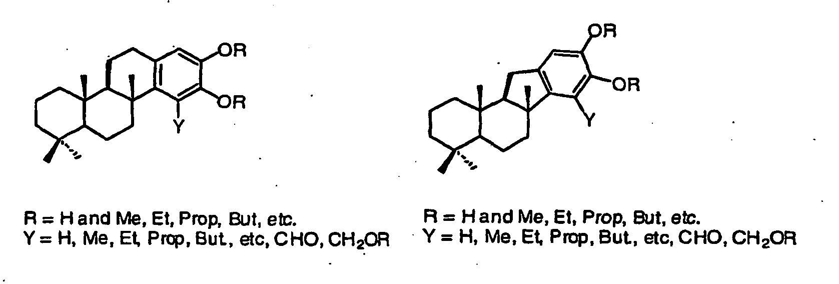

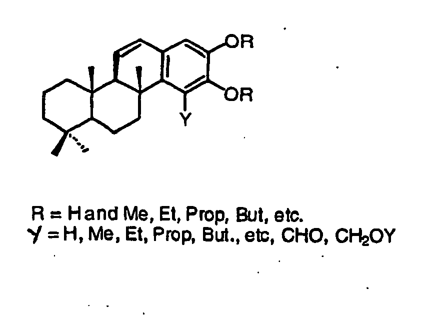

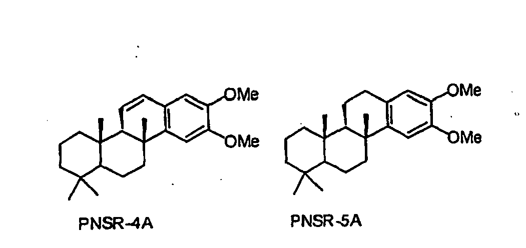

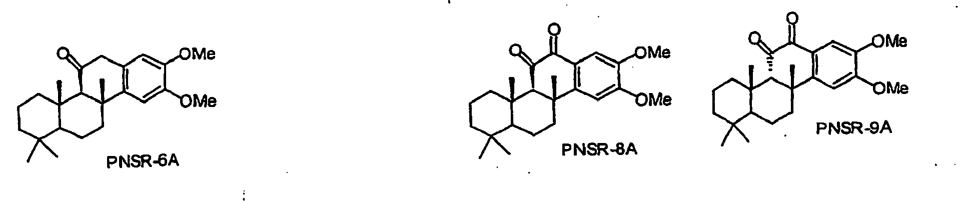

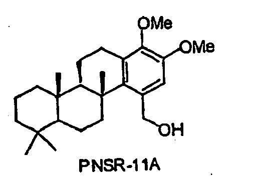

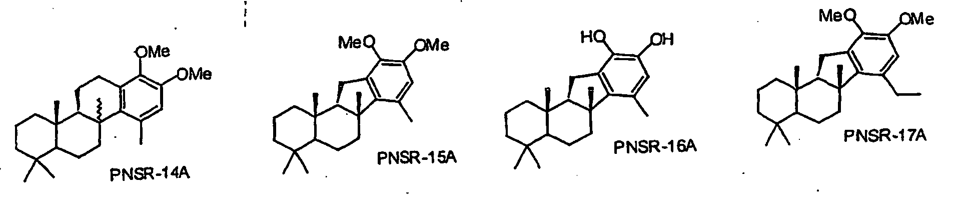

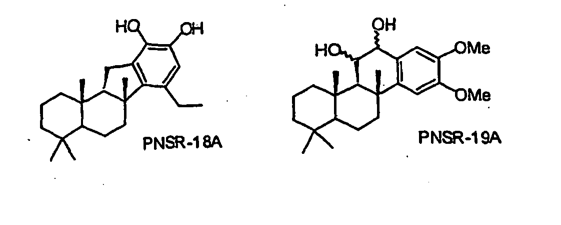

### Sources of Compounds and Assays for Activity

Pelorol may be obtained from natural sources as taught in the prior art and in the Example 1 herein. Solvent fractionation and/or chromatography may be employed. It is also possible to modify pelorol or other available compounds such as chrysene derivatives by known chemical methodologies to add, remove, or replace substituents in order to produce components of Formula **I.** Examples of such derivatization steps as applied to different compounds of Formula **I** are shown in more detail below.

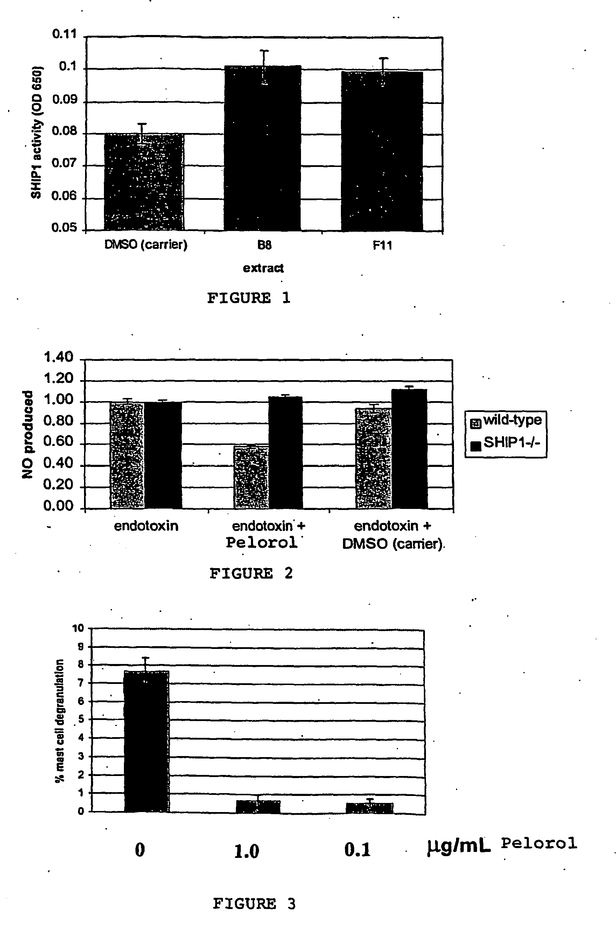

The presence of SHIP 1 modulating compounds in a preparation may be determined by use of a variety of assays, including direct monitoring of a change in activity of SHIP 1 enzyme such as by the methodology disclosed in Example 1 and Figure 1 or by biological assays which may be readily adapted from known procedures, including cell or animal based assays which monitor changes in: nitric oxide production from activated macrophages; IgE induced mast cell degranulation; LPS induced macrophage activation; TNF-α expression or activity. In addition, standard assays for agents which mediate inflammatory activity in living subjects may be employed. Adaptation of these assays is facilitated by the availability of SHIP 1^{-/-} and SHIP 1^{+/-} mice^{15,16} and bone marrow derived macrophages¹⁷. In addition, the availability of anti-SHIP 1 antibodies¹⁸ facilitates use of immunoassay formats. Such assays may also be used to assess activity of compounds prepared by total synthesis, as described herein.

### Total Synthesis of Compounds

A synthetic scheme for making pelorol and other compounds of Formula I is provided herein. Tables (1-2) provide detailed examples of two embodiments of such a synthesis with examples of different compounds of Formula **I** which may be prepared. The compound shown in the Tables that is identical to pelorol except that the ring adjacent the aromatic ring has six members, is termed "homopelorol". Compounds having a six-membered ring are termed "homopelorol analogs". Compounds having a five-membered ring other than pelorol are termed herein, "pelorol analogs".

In the synthesis methods shown in Tables 1 and 2, compounds of Formula IIA shown therein are conveniently based on sclareolide as a starting material. Appropriate derivatives of sclareolide providing desired R₁ - R₄ substituents may be employed. In the aromatic compound of Formula III shown in the Tables, Nu is preferably lithium. X₂ in the starting compound of Formula III is preferably an activating group such as -OMe or - NHAc. X₁ - X₄ may remain as found in the starting material or be appropriately altered to provide the desired substituents for the end product. Protecting groups may be employed on R₁ - R₄ or X₁, X₃, or X₄. An example of derivatization of the ring comprising L' in Formula IA to produce a desired component Q of Formula I is illustrated in Table 2 where oxidation (e.g. by treatment with OsO₄ followed by treatment with an acid such as HCl) is performed to provide a ketone substituent on the ring.

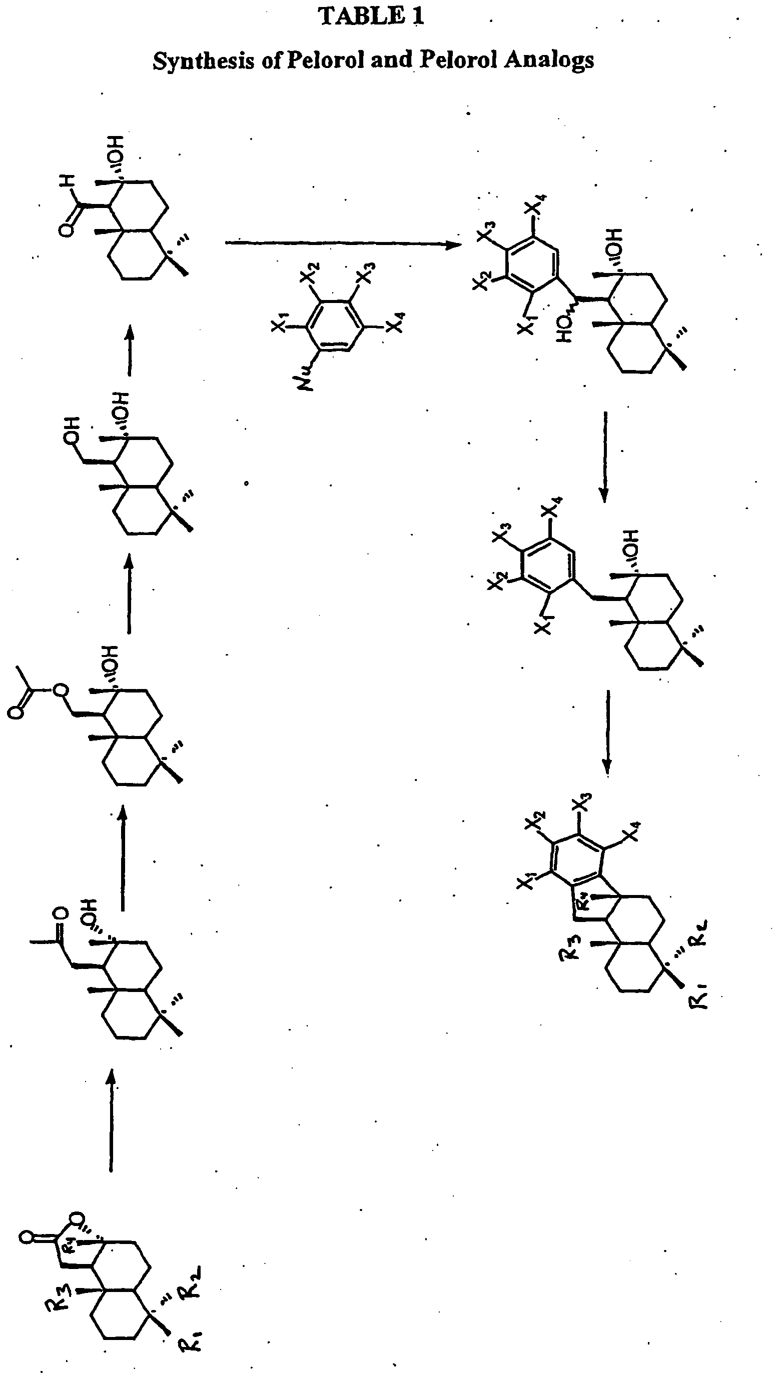

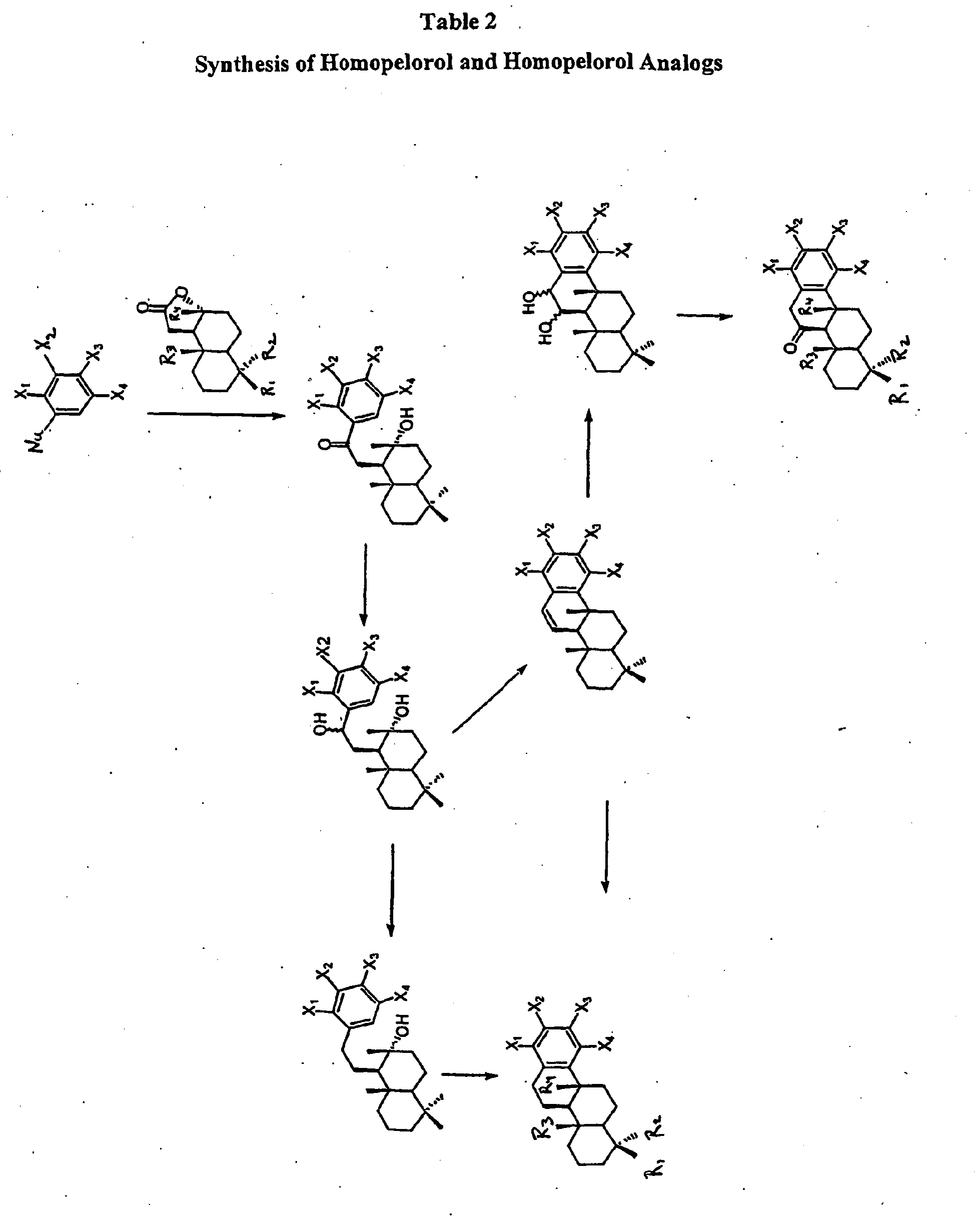

### Pharmaceutical Compositions, Dosages, Administration and Indications

Compounds for use in this invention may be formulated into pharmaceutical compositions in any number of ways, which would be known to a person of skill in the art, all of which are within the scope of the invention. The person of skill in the art may be expected to select appropriate pharmaceutically acceptable salts as well as appropriate pharmaceutically acceptable excipients, diluents, and carriers.

Compounds according to the invention can be provided alone or in combination with other agents (for example, small molecules, peptides, or peptide analogues) in therapeutically- or prophylactically-acceptable amounts, in any pharmaceutically acceptable carrier. Methods well known in the art for making such pharmaceutical formulations are found in, for example, "Remington's Pharmaceutical Sciences" (19th edition), ed. A. Gennaro, 1995, Mack Publishing Company, Easton, PA, incorporated by reference herein. Pharmaceutical formulations according to the present invention may, for example, contain excipients, sterile water, or saline, ethanol, methanol, dimethyl sulfoxide, polyalkylene glycols such as polyethylene glycol, propylene glycol, or other synthetic solvents, oils of vegetable origin, or hydrogenated napthalenes.

Compounds according to the invention may include hydrophobic compounds, for example, compounds that are substantially insoluble in water, but are freely soluble in solvents such as, for example, ethanol, methanol, dimethyl sulfoxide, or chloroform, or combinations thereof. Formulations containing such hydrophobic compounds may be provided using, for example, micelles, which are formed by amphiphilic compounds under certain conditions. In aqueous solutions, micelles are capable of incorporating hydrophobic compounds in their hydrocarbon cores, or within the micelle walls. Hydrophobic compounds may also be provided by solubilization in triglycerides (oils), for example, a digestible vegetable oil. The solubilized hydrophobic compound in the oil phase may be dispersed in an aqueous solution and stabilized using emulsifying agents, if desired. Alternatively, the hydrophobic compound may be provided in oil and delivered, for example, to the gastrointestinal system where bile salts may function as *in vivo* emulsifiers. Hydrophobic compounds may also be provided as microemulsions which, like emulsions, are liquid dispersions of oil and water, but have smaller particles with an oil phase in a micelle-like "core." Hydrophobic compounds according to the invention may also be provided together with a polymeric carrier, for example, a carbohydrate such as starch, cellulose, dextran, cyclodextrin, methylcellulose, or hyaluronic acid, or a polypeptide, such as albumin, collagen, or gelatin. Other modes of formulation of hydrophobic compounds may include liposomes, natural and synthetic phospholipids, or solvents, for example, dimethyl sulfoxide or alcohols.

The pharmaceutical compositions of the invention may be formulated so as to provide controlled release of the active compound(s) over a period of time. Thus, the formulations could contain, for example, an amount of the compound that would be toxic if administered as a single dose, but whose controlled release does not exceed toxic levels. Biocompatible, biodegradable lactide polymer, lactide/glycolide copolymer, or polyoxyethylene-polyoxypropylene copolymers, for example, may be used to control the release of the compounds. Other potentially useful delivery systems for modulatory compounds according to the present invention include ethylene-vinyl acetate copolymer particles, osmotic pumps, implantable infusion systems, and liposomes.

A "therapeutically effective amount" of a compound is an amount effective, at dosages and for periods of time necessary, to achieve the desired therapeutic result using a compound according to the invention. A therapeutically effective amount is also one in which any toxic or detrimental effects of the comopund are outweighed by the therapeutically beneficial effects. A "prophylactically effective amount" of a compound refers to an amount effective, at dosages and for periods of time necessary, to achieve the desired prophylactic result. Typically, a prophylactic dose is used in subjects prior to or at an earlier stage of disease, so that a prophylactically effective amount may be less than a therapeutically effective amount. Amounts considered sufficient will vary according to the specific compound used, the mode of administration, the stage and severity of the disease, the age, sex, weight, and health of the individual being treated, and concurrent treatments.

A preferred range for therapeutically or prophylactically effective amounts of the compounds of the invention may be 0.1 nM-0.1M, 0.1 nM-0.05M, 0.05 nM-15µM or 0.01 nM-10µM. It is to be noted that dosage values may vary with the severity of the condition to be alleviated. For any particular subject, specific dosage regimens may be adjusted over time according to the individual need and the professional judgement of the person administering or supervising the administration of the compositions. Dosage ranges set forth herein are exemplary only and do not limit the dosage ranges that may be selected by medical practitioners. Dosage regimens may be adjusted to provide the optimum therapeutic response. For example, a single bolus may be administered, several divided doses may be administered over time or the dose may be proportionally reduced or increased as indicated by the exigencies of the therapeutic situation.

In general, compounds of the invention should be used without causing substantial toxicity. Toxicity of the compounds of the invention can be determined using standard techniques, for example, by testing in cell cultures or experimental animals and determining the therapeutic index, i.e., the ratio between the LD50 (the dose lethal to 50% of the population) and the LD 100 (the dose lethal to 100% of the population). In some circumstances however, such as in severe disease conditions, it may be necessary to administer substantial excesses of the compositions.

Conventional pharmaceutical practice may be employed to provide suitable formulations or compositions to administer the compounds to patients, depending on the therapeutic or prophylactic objectives. Any appropriate route of administration may be employed, for example, systemic, parenteral, intravenous, subcutaneous, transdermal, transmucosal, intramuscular, intracranial, intraorbital, ophthalmic, intraventricular, intracapsular, intraspinal, intracisternal, intraperitoneal, intranasal, aerosol, topical, surgical, or oral administration. The formulations used may vary according to the chosen route of administration. Thus, for oral administration, the formulations may be in the form of tablets or capsules; for inhalants, the formulations may be in the form of powders, nasal drops, or aerosols; for transmucosal administration, the formulations may be nasal sprays or suppositories; for transdermal administration, the formulations may be creams, ointments, salves, or gels; etc.

Therapeutically effective or prophylactically effective amounts of SHIP 1 modulators and pharmaceutical compositions of this invention may be administered to patients in need of treatment or prophylaxis for cancer (neoplastic diseases), other cell proliferative disorders, inflammatory diseases and immune diseases. Neoplastic diseases include but are not limited to: leukemias, carcinomas, sarcoma, melanomas, neuroblastoma, capillary leak syndrome and hematological malignancies. Diseases with an inflammatory component include, but are not limited to: rheumatoid arthritis, multiple sclerosis, Guillan-Barre syndrome, Crohn's disease, ulcerative colitis, inflammatory bowel syndrome, psoriasis, graft versus host disease, host versus graft, lupus erythematosis, Alzheimer's disease and insulin-dependent diabetes mellitus. Diseases related to inappropriate activation of macrophage-related cells of the reticuloendothelial lineage include osteoporosis.

Pelorol and other compounds having the structure of Formula I exhibit SHIP 1 agonist activity. By activating SHIP 1, such agonists are particularly useful in the treatment of inflammatory diseases such as sepsis/septic shock, colitis, inflammatory bowel syndrome, and those involving macrophage proliferation or activation; neoplastic diseases such as myeloid and lymphoid leukemias; as an immunosuppressive agent such as in transplant rejection; hematopoietic disorders; and for affecting mast cell degeneration such as in the treatment or prevention of allergies.

### Example 1

In a preliminary screen of 150 marine organism extracts, extracts which activated SHIP 1 in an enzyme assay were identified. Assay-guided fractionation of one of these extracts resulted in the identification of the active compound as being pelorol (Figure 1). The origin and processing of the extracts which tested positive in the screen and the nature of the assay were as follows.

Specimens of the brownish sheet sponge *Dactylospongia elegans* (order Dictyoceratida, family Spongiidae) were collected by hand using SCUBA at a depth of 5-10 m from a protected overhang in Rasch Passage on the outer reef of Madang Lagoon, Papua New Guinea, in January 1995. Freshly collected sponge was frozen on sight and transported to Vancouver, Canada over dry ice. The sponge was identified and for verification, a voucher sample was placed in the Zoological Museum of Amsterdam (ZMA POR. 15986). The frozen sponge (120 g) was cut into small pieces, immersed in and subsequently extracted repeatedly with MeOH (3 x 250 mL). The combined methanolic extracts were concentrated *in vacuo* and then partitioned between EtOAc (4 x 100 mL) and H₂O (300 mL). The combined EtOAc extract was evaporated to dryness *in vacuo* to yield 490 mg of a brownish purple oil, found to contain pelorol.

The assay was performed in 96-well microtitre plates. SHIP 1 enzyme was produced with a hemagglutinin and a hexahistidine tag, from a mammalian expression vector. The His tag was employed to enhance purification. SHIP 1 enzyme (10ng) was incubated with extract or DMSO for 15 minutes at room temperature before addition of 200 M inositol-1,3,4,5-tetrakisphosphate. The reaction was allowed to proceed for 20 minutes at 37 degrees C. The amount of inorganic phosphate released was then assessed by the addition of malachite green reagent followed by an absorbance measurement at 650 nm.

### Example 2

Pelorol was prepared according to the following scheme, under the specific conditions described below.

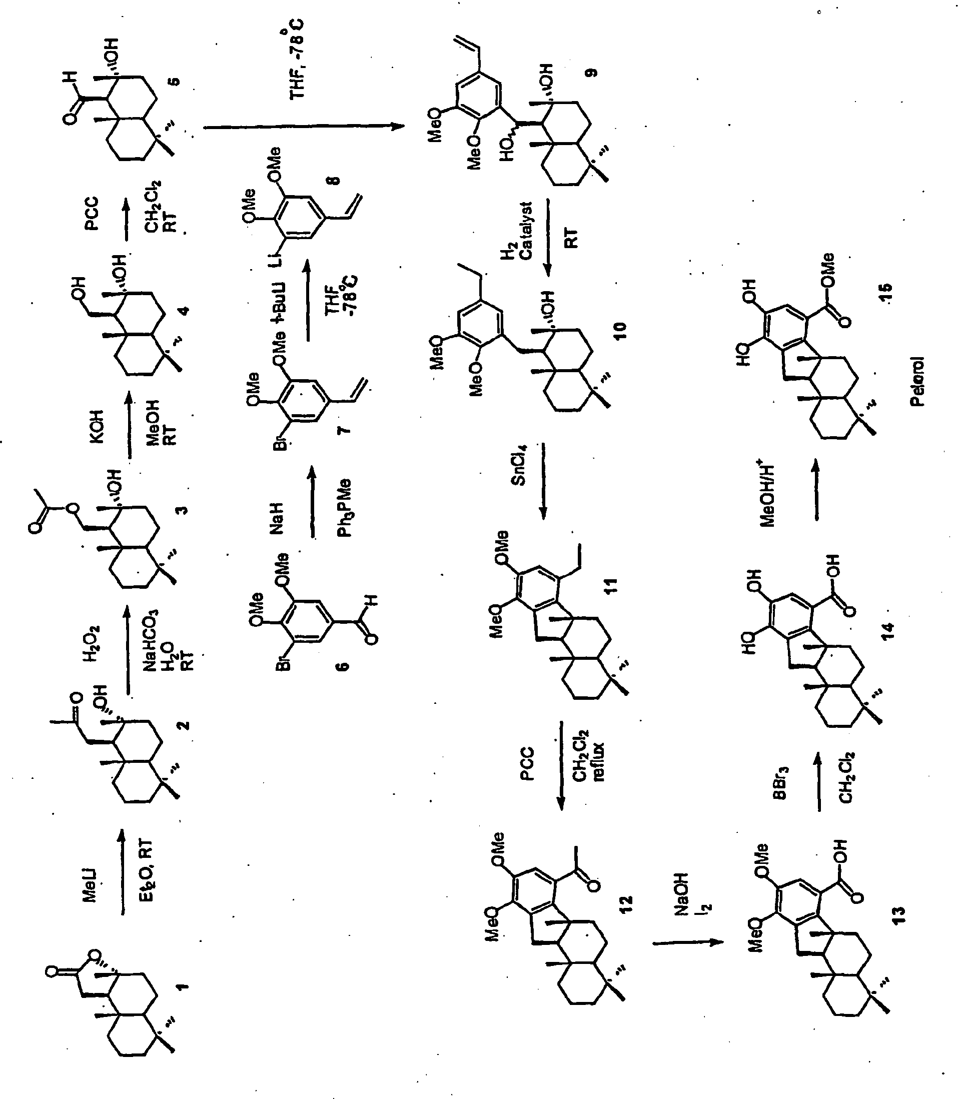

To a stirred solution of **1** (1.00g, 3.99mmol) in anhydrous Et₂O (30mL) was added a freshly prepared 1.6M solution of MeLi in Et₂O (3mL, 4.8mmol) in portions for 10 min at r.t. and stirring was continued for another 5 min. The mixture was then treated with 10% HCl (2mL), then transferred to a funnel and extracted with ethereal repeatedly. The combined extracts was washed with NaHCO₃ and H₂O, dried (MgSO₄), filtered and concentrated. The residue was column chromatographed with hexane/Et₂O (6:4) to give 0.74g (70%) of **2**.

To a stirred, cooled (ice bath) solution of (CF₃CO)₂O (9mL, 63.85mmol) in CH₂Cl₂ (40mL) was added 50% aq H₂O₂ (1.8L, 31.66mmol) and the mixture was allowed to stand in an ice bath for 10min. All subsequent operations were performed at r.t. The solution was treated with solid NaHCO₃ (5.40g, 64.28mmol) for 2 min and after stirring the mixture for **8** min, a solution of 2 (1.80g, 6.76mmol) in CH₂Cl₂ (54mL) was added. The resulting mixture was stirred for 30min and then, after addition of H₂O (10mL), was treated with solid NaHCO₃ in portions for 45 min until the pH reached 7. Finally, the mixture was extracted with Et₂O. The combined extracts were washed with NaHCO₃, H₂O and dried (MgSO₄), filtered and concentrated to give pure **3**.

Compound **3** (1g, 3.6mmol) was dissolved in 10% solution of KOH in MeOH (1mL, 1.78mmol) at 0°C. The resulting mixture was stirred for 10min. After addition of H2O, the solution was extrated with Et2O. The extrated was washed with H₂O, dried (MgSO₄), filtered and concentrated to give 0.8g of **4**.

In an oven dried , N₂ flushed 100mL round bottom flask equipped with a magnetic stirring bar was placed 3.24g(15mmol) of PCC, 30 mL of CH₂Cl₂ and 2.4 g (10mmol) of **4**. The mixture was well stirred at r.t. for 2hrs and was quenched by adding 30mL of Et₂O. The resulting solution was filtered through a thick pad of silica gel and concentrated to give a residue. The residue was column chromatographed with hexane/EA (8:2) to give 1.6g (67%) of **5**.

Sodium hydride (24.6mg, 0.82mmol, 80% oil dispersion) and dry THF (5mL) were added to a dry flask equipped with a condenser and dry N₂ flow. To this suspension was added methyl triphenyl phosphonium bromide (0.146g, 0.41mmol) and the mixture was stirred for 10min. Then **6** (100mg, 0.41mmol) in THF (2mL) was added and the mixture was gently reflux for 2h. The reaction was quenched by adding 2mL of methanol and then extracted with Et₂O. After usual work up treatment. 94.3mg of 7 was afforded.

A 1.6M solution of tBuLi in pentane (1.74mL, 2.79mmol) was added slowly to a stirred solution of **7** (612.6mg, 2.52mmol) in dry THF (20mL) at -78°C. After stirring for 30min, a solution of **5** (300mg, 1.26mmol) in dry THF (5mL) was added. The mixture was further stirred at -78°C for 2hrs. Then H₂O (10mL) was added and the mixture was extracted with Et₂O (120mL twice). The combined Et₂O extracts were washed with sat.brine, dried (MgSO₄) and concentrated to give a residue, which was chromatographed on NP Sepak^{™} to give 280mg (55%) of **9**.

A solution of **9** (40mg, 0.1mmol) in EA (5mL) was hydrogenated over 10% Pd/C (50mg) under an atmosphere of hydrogen at r.t. over night. Filtration and concentration gave 37mg (96%) of **10**.

To a stirred solution of **10** (38.8 mg, 0.1mmol) in CH₂Cl₂ (10 mL), SnCl₄ (0.1mL) was added slowly at -20°C under argon for 2 min. The resulting mixture was further stirred for 20min and then diluted with CH₂Cl₂ (20mL) and poured into ice. The aqueous phase was extracted with CH₂Cl₂ twice (20mL) and combined the extracts, washed with saturated NaHC03, saturated brine and dried over MgSO₄. Evaporation to afford **11** (28mg, 76%).

PCC (41.6mg, 0.92mmol) was added to **11** (7.4mg, 0.02mmol) dissolved in 2mL of CH₂Cl₂. The mixture was stirred at gentle reflux for 24 hrs under Argon. The reaction was diluted with Et₂O (20mL) and the resulting dark solution was filter through a NP Sepak^{™}. Concentration of the filtrates and further purification afford 1.5mg (20%) of **12.**

1.5mg of **12** was dissolved and stirred in 2mL of NaOH(10%) solution (containing 0.5mL THF). 5mg Iodine is added subsequently and the mixture was further stirred for 20min and acidified by adding 3mL of 10% H₂SO₄. The solution was extracted with 50mL of Et₂O, washed with saturated brine and concentrated to afford a residue **13.**

38.6mg (0.1mmol) of **13** was stirred in CH₂Cl₂ (1mL) under Argon. BBr₃ in CH₂Cl₂ (2.0mL 1M) was added, and stirring was continued for 1.5h. The mixture was then poured into H₂O and extracted with CH₂Cl₂ (50mL). The combined extracts were then dried over MgSO₄, filtered and concentrated. The residue was purified by NP Sepak^{™} (hexane:EA=7:3) to afford **14** (25mg, 70%).

35.8mg (0.1mmol) of **14** was dissolved in MeOH (2mL) containing 5% H₂SO₄. Stirring was continued for 2hr and the mixture was extracted with Et₂O, dried over MgSO₄ and concentrated to afford **15.**

### Example 3

The pelorol analog PNSR-15A was synthesized following the methodologies described in Example 3 using the following scheme.

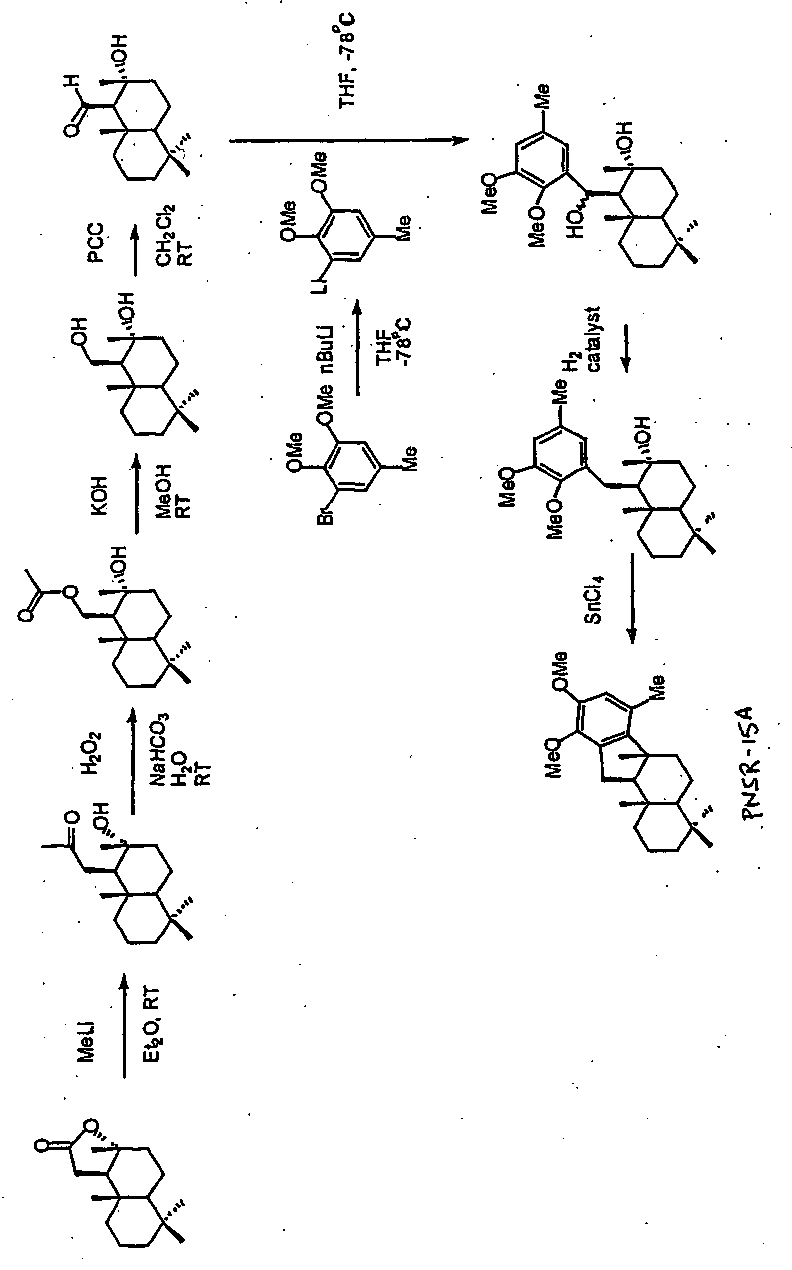

### Example 4

The homopelorol analog PNSR-4A was synthesized by the methodologies described above and according to the following scheme.

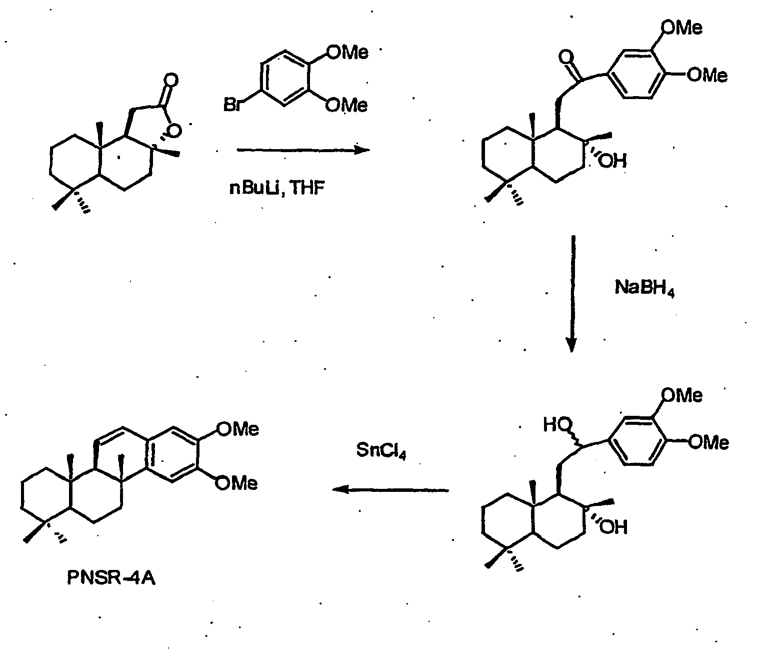

### Example 5

The homopelorol analog PNSR-14A was synthesized by the methodologies described above and according to the following scheme.

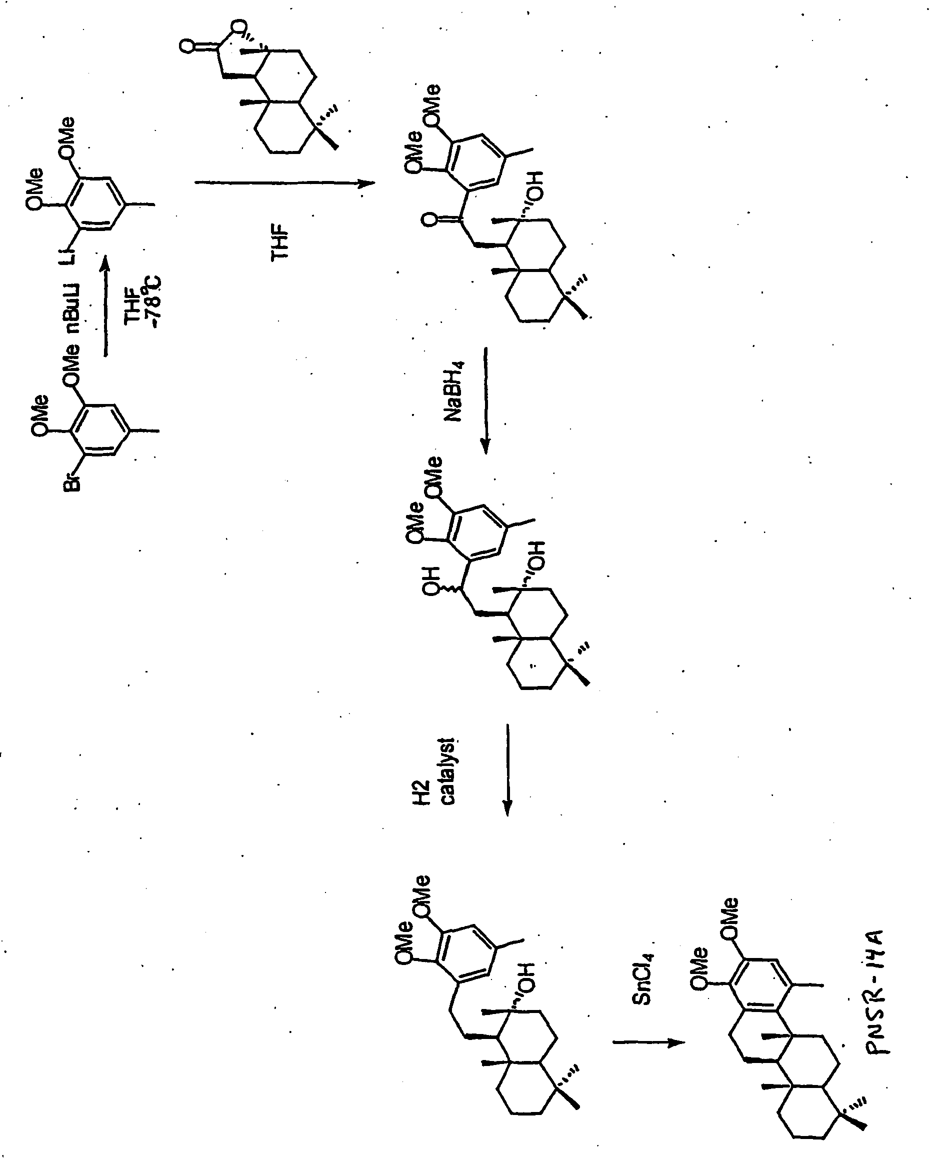

### Example 6

Homopelorol may be synthesized according to the following scheme based on the preceding examples and following the methodologies described above.

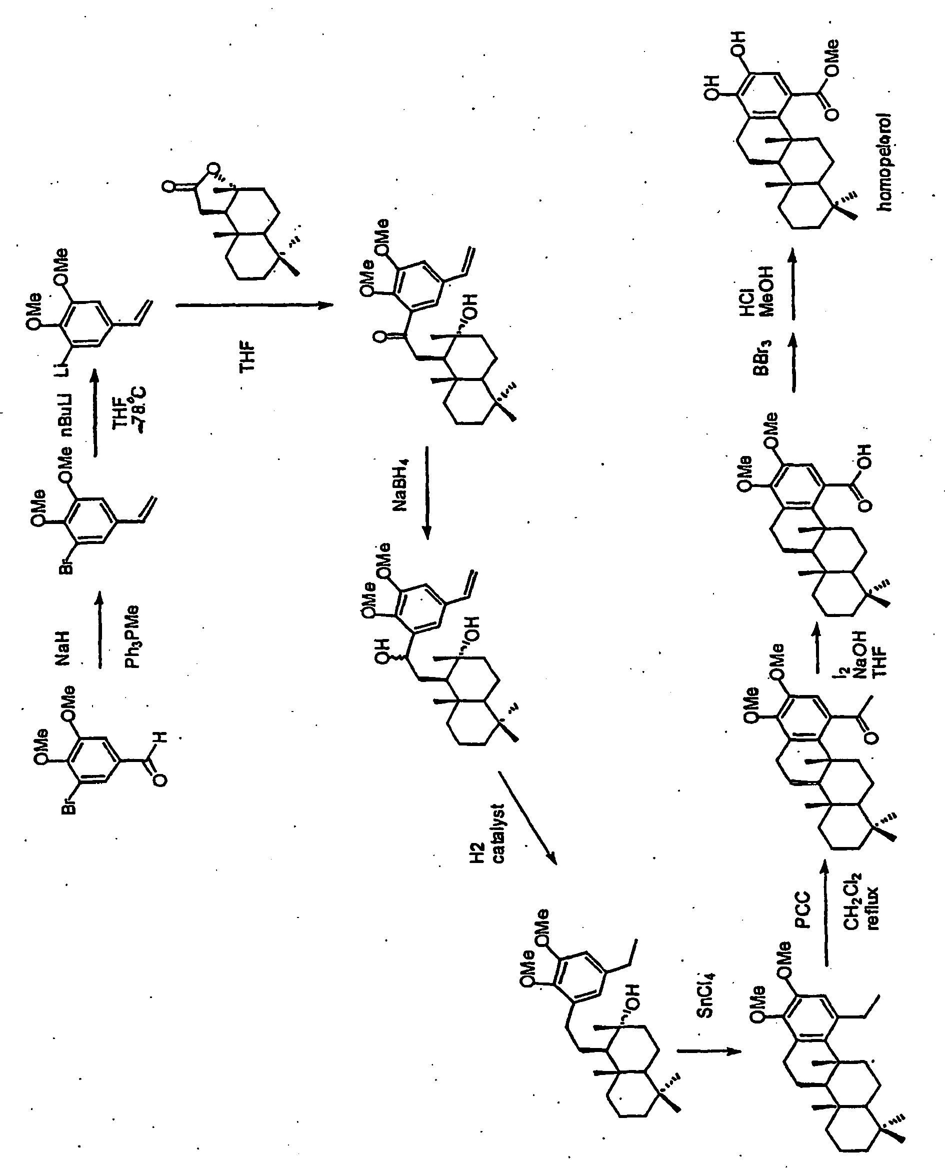

### Example 7

In addition to causing an increase in activity in the SHIP 1 enzyme assay described for Figure 1, agonist compounds of Formula I exhibit anti-inflammatory actions on macrophages and mast cells in intact cell-based assays, inhibit nitric oxide production from endotoxin activated wild-type macrophages and exhibit anti-inflammatory actions on live subjects. Results obtained for pelorol in NO release and mast cell activation assays are shown in Figures 2 and 3, respectively. Inhibition of NO release was not observed in SHIP 1 -/- macrophages. Pelorol significantly reduced IgE induced mast cell degranulation.

Procedures used in the cell and animal based assays are described below. Results for pelorol and various analogs within Formula I are shown in Table 3, including results using the enzyme assay described in Example 1.

For the NO release assay, wild-type or SHIP 1 -/- macrophage cells were aliquoted into microtitre plates (5x10⁴/well) and activated with 1 g/mL endotoxin (LPS) in the presence or absence of test compound or DMSO carrier. The cells were incubated at 37°C, 5% CO₂ for 24 hours and the culture supernatant was removed for NO determination using the Griess reagent. Alternatively, J774.1 a macrophage cells were treated with 10 µg/ml of test compound dissolved in DMSO for 40 minutes prior to the addition of LPS. Culture supernatants were collected after 24 hr. for determination of NO concentration using the Griess reagent.

For the mast cell activation assay, bone marrow derived mast cells were incubated at 4°C with anti-DNP IgE for 1 hr. They were then washed twice with 23°C Tyrode's buffer, and incubated in the presence of test compound or vehicle control for 30 minutes before a 15 minute treatment with DNP-human serum albumin. The degree of degranulation was determined by measuring the release of β-hexosaminidase.

For the macrophage TNF-α production assay, J774.1a macrophage cells were treated with 10 µg/mL of test compound dissolved in cyclodextrin for 40 minutes prior to the addition of 100ng/mL LPS. Culture supernatants were collected after 2 hr and 5 hr for TNF-α determination by ELISA.

The mouse ear edema (Evans Blue) assay is a standard model for allergic inflammation. Mice were passively sensitized by intravenous injection of monoclonal anti-DNP IgE antibody. 24 hours later, 10 µg test compound (right ears) in 20 µl DMSO:Methanol (1:3) or vehicle alone (left ears) were applied 20 minutes followed by application of the inducing agent [20 µl of 0.15% DNFB in acetone:olive oil (4:1)]. Mice were then injected intravenously with 300 µl 1% Evans Blue. Vascular permeability was measured at 1 hr after application of the inducing agent by visual inspection and quantification of Evans Blue extravasation in the ear. To quantify the Evans Blue content, ears were harvested at 1 hr post DNFB treatment and Evans Blue was extracted by incubation in formamide at 37°C for 24 hr and quantified by spectophotometry at 620 nm. Ears pretreated with carrier alone mounted a prompt anaphylactic reaction in response to DNFB challenge. In contrast, SHIP 1 agonists showed a clear inhibition of vascular permeabilization as shown by decreased Evans Blue extravasation.

The mouse ear edema (lymphocyte infiltration assay) is a contact hypersensitivity or ear inflammation model and is a standard *in vivo* model for human allergy. Contact hypersensitivity consists of an initial sensitizing phase and an elicitation phase. The latter phase occurs when the epidermal cells encounter a particular antigen to which they have previously been exposed and is characterized by localized immune cell infiltration, inflammation, and edema. In this assay, female 4 week old (20g) Balb/c mice were sensitized to the haptenizing agent, 2,4-dinitrofluorobenzene (DNFB) by shaving their abdominal region with an electric razor before applying 25 µl of 0.5% DNFB in acetone:olive oil (4:1, v/v) to the abdominal wall for two consecutive days. Four days after the second application, mice were lightly anesthesized with halothane before being challenged (treated) epicutaneously on each side of the right and left ear with 10 µl of 0.2% DNFB. All mice received a 500 µl intraperitoneal (i.p.) injection of [³H]-methyl thymidine in sterile saline (1 µCi/g body weight) 24 hours before epicutaneous challenge with DNFB. Thirty minutes prior to DNFB challenge, the right and left ears were pretreated with test compound in DMSO:methanol (1:3, v/v) or vehicle alone, respectively. Twelve hours following DNFB challenge, mice were sacrificed by CO₂ asphyxiation and 8 mm diameter plugs were taken from each ear and digested in 500 µl solvable^{™} at 60°C. for 10-12 hours. Samples were decolourized by the addition of H₂O₂ and analyzed for radiolabelled leukocyte infiltrates by standard liquid scintillation counting.

The colitis assay is based on determining whether a test compound protects mice from TNBS (trinitrobenzene sulfonic acid) induced inflammation. Test compound (10mg/kg) or vehicle control was injected intraperitoneally into mice just prior to a TNBS enema administration. After 2 days, the colons of the vehicle treated mouse were severely inflamed while the SHIP 1 agonist treated mouse had no signs of inflammation.

**TABLE 3**

| | SHIP enzyme assay | Macrophage NO production | Macrophage TNFα production | Mast cell activation | Mouse ear edema (evans blue assay) | Mouse ear edema (leukocyte infiltration assay) | colitis |
|---|---|---|---|---|---|---|---|
| Pelorol | +++ | +++ | +++ | +++ | +++ | ND | +++ |
| Dimethoxypeloro | + | +++ | ND | ND | ND | ND | ND |
| PNSR-4A | insol | ND | ND | ND | +++ | ND | ND |
| PNSR-15A | ND | ND | ND | ND | ND | ++ | ND |
| PNSR-16A | ND | ND | +++ | ND | ND | ++ | ND |
| PNSR-17A | ND | ND | ++ | ND | ND | ND | ND |
| PNSR-18A | ND | ND | +++ | ND | ND | ND | ND |

Although the foregoing invention has been described in some detail by way of illustration and example for purposes of clarity of understanding, it will be readily apparent to those of skill in the art in light of the teachings of this invention that changes and modification may be made thereto without departing from the spirit or scope of the appended claims. All patents, patent applications and publications referred to herein are hereby incorporated by reference.

### References

1. Huber, M. et al. (1999) Prog Biophys Mol Biol 71:423.
2. Sattler, M. et al. (1999) Mol Cell Biol 19:7473.
3. Lui, Ling et al. (2001) Blood 98:1225 (Abstract No. 1225).
4. Kwak, J.H. et al. (2000) J. Nat. Prod 63:1153-56.
5. Goclik, E. et al. (2000) J. Nat. Prod 63:1150-52.
6. Ishihara, Kazuaki et al.(2001) J. of the American Chem. Soc. 123:1505-1506.
7. Ishihara, Kazuaki et al. (2002) J. of the American Chem. Soc.124:3647-3655.
8. Rosales, Viale et al. (2002) J. of Organic Chem. 67:1167-1170.
9. Corey, Elias J. et al. (1998) Angewante Chemie, Intl. Ed. 37:1126-1128.
10. Boreham, Christopher J. et al. (1995) Organic Geochemistry 23:461-6.
11. Freeman, Katherine H. et al. (1994) Organic Geochemistry 21:1037-1049.
12. Schaeffer, Phillipe et al. (1994) Angewante Chemie 106:1235-8.
13. Harrington, Scott R. et al. (1994) Tetrahedron 50:9229-54.
14. Registry Number 112299-69-1
15. Helgason, C. D. et al. (1998) Genes Dev. 12:1610.
16. Helgason, C. D. et al. (2000) J. Exp. Med. 191:781.
17. O'Farrell, A. M. et al. (2000) J. Immunol. 164:4607.
18. Damen, J. E. et al. (1998) Blood 92:1199.

## Claims

1. A compound of Formula I or a salt thereof, 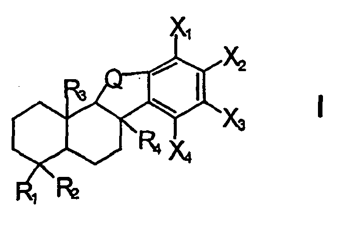 wherein;
R₁ and R₂ are independently selected from the group consisting of: -CH₃, -CH₂CH₃, -CH₂OH, -CH₂OR', -CHO, -CO₂H, and -CO₂R';
R₃ and R₄ are independently selected from the group consisting of: H, -CH₃, -CH₂CH₃, -CH₂OH, -CH₂OR', -CHO, -CO₂H, and -CO₂R';
Q is a carbon skeleton selected from the group consisting of: -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂-, and -CY₁Y₂CY₃Y₄CY₅Y₆-; where Y₁, Y₂, Y₃, Y₄, Y₅, and Y₆ are independently selected from the group consisting of: H, F, Br, Cl, I, OH, OR', and SH; or any one group of Y₁/Y₂, Y₃/Y₄, and Y₅/Y₆ may be =O; or Y₁/Y₃ may form an epoxide; and, at least one of Y₁, Y₂, Y₃, Y₄, Y₅ and Y₆ when present, is not H;
X₁, X₂, X₃, and X₄ are independently selected from the group consisting of: H, R, OH, -OR, -CO₂H, -CO₂R', F, Br, Cl, I, -CN, -SO₃H, -OSO₃H, NO₂, NH₂, -NHR, and -NR₂; where R is a linear, branched, or cyclic, saturated or unsaturated one to ten carbon alkyl group that is unsubstituted or is substituted with one or more of: OH, =O, SH, F, Br, Cl, I, NH₂, -NHR', -NR'₂, NO₂, -CO₂H, -CO₂R', and epoxide;
and R' is a linear, branched, or cyclic, saturated or unsaturated one to ten carbon alkyl group that is unsubstituted or substituted with one or more of: OH, =O, SH, F, Br, Cl, I, NH₂, NHR", -NR"₂, NO₂ and -CO₂H where R" is a linear, branched, or cyclic, saturated or unsaturated one to ten carbon alkyl group;
providing that the compound does not have the precise structure of pelorol or any one of the group of structures consisting of:

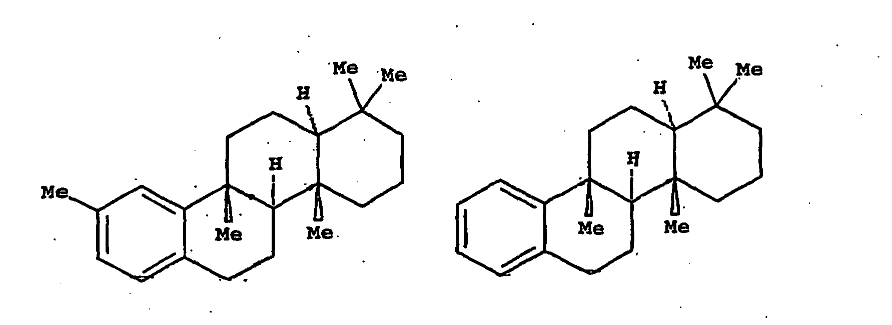

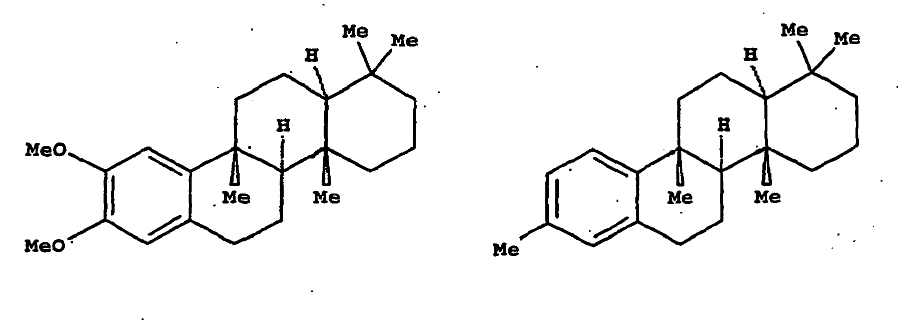

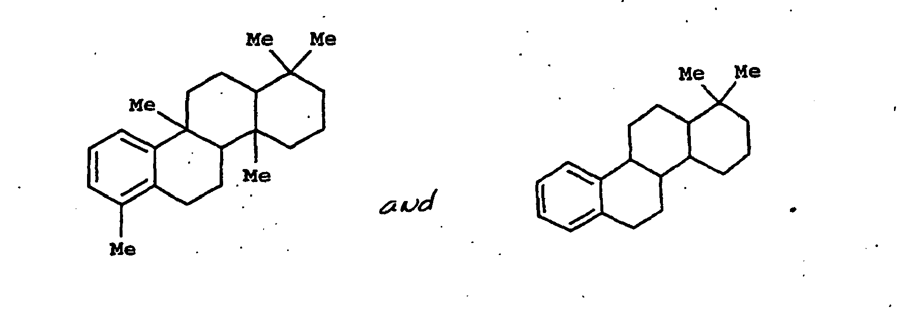

2. The compound of claim 1, wherein Y₁ - Y₆ are independently selected from H, F, Br, Cl and I.

3. The compound of claim 1, wherein Q is -CH₂-.

4. The compound of claim 1, wherein Q is -CH₂CH₂-.

5. The compound of claim 1, wherein Q is -CH=CH-, -CH₂CH₂CH₂-, or -CH=CHCH₂-.

6. The compound of any one of claims 1-5, wherein R₁ is methyl, ethyl, -CH₂OH, or -CH₂OR'.

7. The compound of any one of claims 1-6, wherein R₂ is methyl, ethyl, -CH₂OH, or -CH₂OR'.

8. The compound of any one of claims 1-7, wherein R' in R₁ is limited to methyl, ethyl, propyl or butyl.

9. The compound of any one of claims 1-8, wherein R' in R₂ is limited to methyl, ethyl, propyl or butyl.

10. The compound of claim 8 or 9, wherein R' is limited to methyl or ethyl.

11. The compound of any one of claims 1-10, wherein X₁ is H, OH, R, OR, -CONH₂, -CONHR', or -COR'₂.

12. The compound of any one of claims 1-11, wherein X₂ is H, OH, R, OR, -CONH₂, -CONHR', or -COR'₂.

13. The compound of any one of claims 1-12, wherein X₃ is H, OH, R, OR, -CONH₂, -CONHR', or -COR'₂.

14. The compound of any one of claims 1-13, wherein R and R' in one or more of X₁, X₂, and X₃ are limited to methyl, ethyl, propyl and butyl.

15. The compound of any one of claims 1-10, wherein X₁ is H, OH, or -OCH₃.

16. The compound of any one of claims 1-10 and 15, wherein X₂ is H, OH, or OCH₃.

17. The compound of any one of claims 1-10 and 15, wherein X₂ is H, OCH₃, or -NHOCH₃.

18. The compound of any one of claims 1 -10,15,16, and 17, wherein X₃ is H, OH, or OCH₃.

19. The compound of any one of claims 1-18, wherein X₄ is H, R, OH, OR, CO₂H or CO₂R'.

20. The compound of any one of claims 1-19, wherein R and R' in X₄ are limited to methyl, ethyl, propyl or butyl.

21. The compound of any one of claims 1-18, wherein X₄ is H, R, OH, OCH₃, -CO₂H or -CO₂CH₃.

22. The compound of claim 1 which is homopelorol of the formula 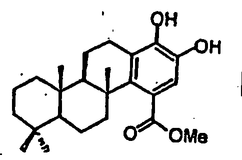 .

23. The compound of claim 1 which is dimethoxypelorol of the formula 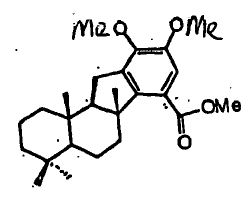 .

24. The compound of claim 1 which is PNSR-4A of formula:

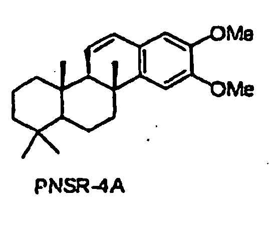

25. The compound of claim 1 which is PNSR-15A of formula:

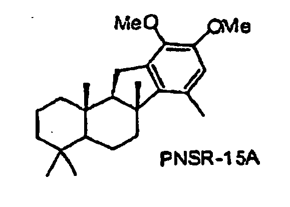

26. The compound of claim 1 which is PNSR-16A of formula:

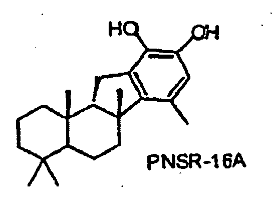

27. The compound of claim 1 which is PNSR-17A of formula:

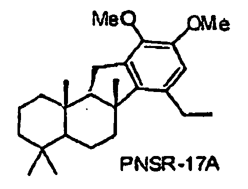

28. The compound of claim 1 which is PNSR-18A of formula:

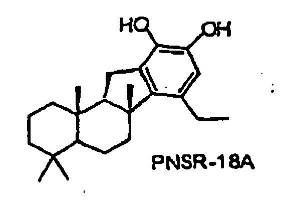

29. The compound of any one of claims 1-28, having the configuration S, R, R, S at C-5, C-8, C-9 and C-10 respectively.

30. The compound of any one of claims 1-28, having the configuration R, S, S, R at C-5, C-8, C-9 and C-10 respectively.

31. The compound of any one of claims 1-30, for use as a modulator of SHIP 1 activity.

32. The compound of claim 31, wherein the compound is an agonist of SHIP 1 activity.

33. A compound according to any one of claims 1-32 for use in a method of treatment or prevention of an inflammatory, neoplastic, hematopoietic or immune disorder or condition.

34. A method of making a compound of Formula IA in which R₁ - R₄, X₁, X₃ and X₄ are as defined in claim 1, 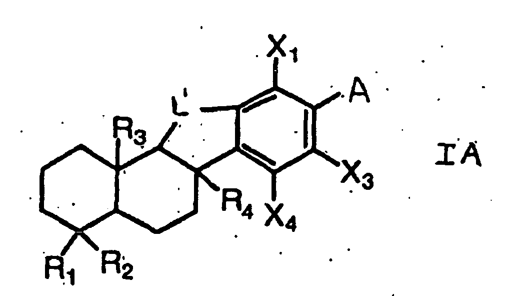 wherein, L' is a C₁ - C₃ saturated or unsaturated alkyl linking group; and A is an activating group; comprising reacting a compound of Formula IIB: 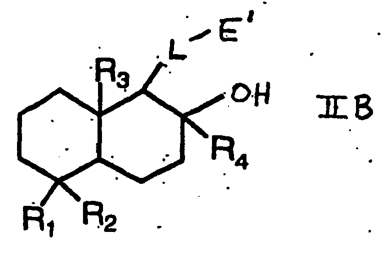 in which L is absent or is a C₁ - C₃ saturated or unsaturated alkyl linking group and E' is an electrophilic reactive group; with a compound of Formula III 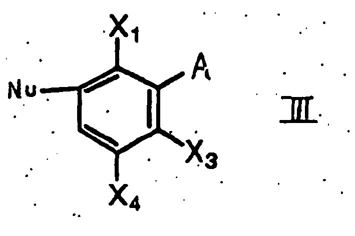 in which Nu is a group that renders the compound of Formula III nucleophilic at Nu, followed by optional reduction and by hydrolysis, to produce a compound of Formula IV 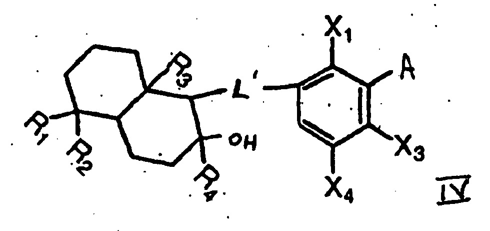 and condensing the compound of Formula IV to produce a compound of Formula IA.

35. The method employing the reaction scheme of claim 34; wherein the compounds of Formula IIB has the structure: 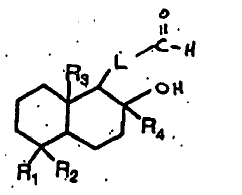 or wherein the compounds of formula IIB are replaced by those of formula : 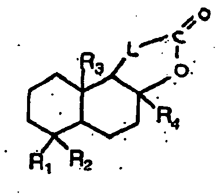 .

36. The method of claim 35, wherein the compounds of Formula IIB has the structures

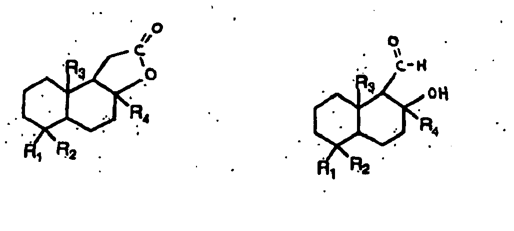

37. The method of any one of claims 35-36, wherein the compound of Formula IIB is sclareolide or is derived from sclareolide.

38. The method of any one of claims 34-37, wherein Nu is lithium.

39. The method of any one of claims 34-39, wherein A is OCH₃ or NHOCH₃.

40. A pharmaceutical composition comprising a pharmaceutically acceptable carrier and one or more compounds of Formula I or pharmaceutically acceptable salts thereof, 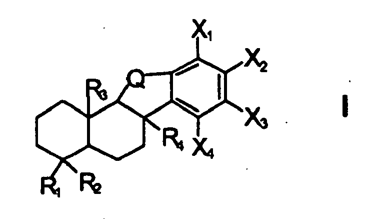 wherein;
R₁ and R₂ are independently selected from the group consisting of: -CH₃, -CH₂CH₃, -CH₂OH, -CH₂OR', -CHO, -CO₂H, and -CO₂R';
R₃ and R₄ are independently selected from the group consisting of: H, -CH₃, -CH₂CH₃, -CH₂OH, -CH₂OR', -CHO, -CO₂H, and -CO₂R';
Q is a carbon skeleton selected from the group consisting of: -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂-, and -CY₁Y₂CY₃Y₄CY₅Y₆-; where Y₁, Y₂, Y₃, Y₄, Y₅, and Y₆ are independently selected from the group consisting of: H, F, Br, Cl, I, OH, OR', and SH; or any one group of Y₁/Y₂, Y₃/Y₄, and Y₅/Y₆ may be =O; or Y₁/Y₃ may form an epoxide; and, at least one of Y₁; Y₂, Y₃, Y₄, Y₅ and Y₆ when present, is not H;
X₁ X₂, X₃, and X₄ are independently selected from the group consisting of: H, R, OH, -OR, -CO₂H, -CO₂R', F, Br, Cl, I, -CN, -SO₃H, -OSO₃H, NO₂, NH₂, -NHR, and -NR₂;
where R is a linear, branched, or cyclic, saturated or unsaturated one to ten carbon alkyl group that is unsubstituted or is substituted with one or more of: OH, =O, SH, F, Br, Cl, I, NH₂, -NHR', -NR'₂, NO₂, -CO₂H, -CO₂R', and epoxide;
and R' is a linear, branched, or cyclic, saturated or unsaturated one to ten carbon alkyl group that is unsubstituted or substituted with one or more of: OH, =O, SH, F, Br, Cl, I, NH₂, -NHR", -NR"₂, NO₂ and -CO₂H where R" is a linear, branched, or cyclic, saturated or unsaturated one to ten carbon alkyl group;
wherein the one or more compounds of Formula I is not solely pelorol.

41. The pharmaceutical composition of claim 40, comprising pelorol.

42. The pharmaceutical composition of claim 40 or 41, comprising a compound according to any one of claims 1-32.

43. A pharmaceutical composition according to any one of claims 40-42 for use in a method of prophylaxis or treatment of an immune, hematopoietic, inflammatory or neoplastic disorder.

44. A pharmaceutical composition comprising a pharmaceutically acceptable carrier and one or more compounds of Formula I or pharmaceutically acceptable salts thereof for use in a method of prophylaxis or treatment of an immune, hematopoietic, inflammatory or neoplastic disorder, wherein said compound of formula I is: 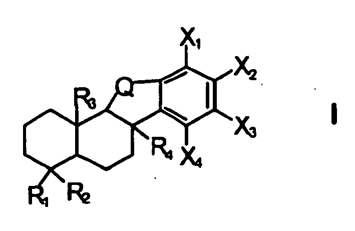 wherein;
R₁ and R₂ are independently selected from the group consisting of: -CH₃, -CH₂CH₃, -CH₂OH, -CH₂OR', -CHO, -CO₂H, and -CO₂R';
R₃ and R₄ are independently selected from the group consisting of: H, CH₃, -CH₂CH₃, -CH₂OH, -CH₂OR', -CHO, -CO₂H, and -CO₂R';
Q is a carbon skeleton selected from the group consisting of -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂-, and -CY₁Y₂CY₃Y₄CY₅Y₆-; where Y₁, Y₂, Y₃, Y₄, Y₅, and Y₆ are independently selected from the group consisting of: H, F, Br, Cl, I, OH, OR', and SH; or any one group of Y₁/Y₂, Y₃/Y₄, and Y₅/Y₆ may be =O; or Y₁/Y₃ may form an epoxide; and, at least one of Y₁, Y₂, Y₃, Y₄, Y₅ and Y₆ when present, is not H;
X₁, X₂, X₃, and X₄ are independently selected from the group consisting of: H, R, OH, -OR, -CO₂H, -CO₂R', F, Br, Cl, I, -CN, -SO₃H, -OSO₃H, NO₂, NH₂, -NHR, and -NR₂;
where R is a linear, branched, or cyclic, saturated or unsaturated one to ten carbon alkyl group that is unsubstituted or is substituted with one or more of: OH, =O, SH, F, Br, Cl, I, NH₂, -NHR', -NR'₂, NO₂, -CO₂H, -CO₂R', and epoxide;
and R' is a linear, branched, or cyclic, saturated or unsaturated one to ten carbon alkyl group that is unsubstituted or substituted with one or more of: OH, =O, SH, F, Br, Cl, I, NH₂, -NHR", -NR"₂, NO₂ and -CO₂H where R" is a linear, branched, or cyclic, saturated or unsaturated one to ten carbon alkyl group.

## Patentansprüche

1. Verbindung der Formel I oder ein Salz davon, 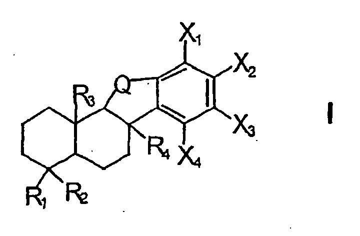 worin:
R₁ und R₂ unabhängig voneinander aus der aus -CH₃, -CH₂CH₃, -CH₂OH, -CH₂OR', -CHO, -CO₂H und -CO₂R' bestehenden Gruppe ausgewählt sind;
R₃ und R₄ unabhängig voneinander aus der aus H, -CH₃, -CH₂CH₃, -CH₂OH, -CH₂OR', -CHO, -CO₂H und -CO₂R' bestehenden Gruppe ausgewählt ist;
Q ein Kohlenstoffskelett ist, das aus der aus -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂- und -CY₁Y₂CY₃Y₄CY₅Y₆- bestehenden Gruppe ausgewählt ist; wobei Y₁, Y₂, Y₃, Y₄, Y₅ und Y₆ unabhängig voneinander aus der aus H, F, Br, Cl, I, OH, OR' und SH bestehenden Gruppe ausgewählt sind; oder eine beliebige der Gruppen Y₁/Y₂, Y₃/Y₄ und Y₅/Y₆ gegebenenfalls =O ist; oder Y₁/Y₃ gegebenenfalls ein Epoxid bildet; und zumindest eines von Y₁, Y₂, Y₃, Y₄, Y₅ und Y₆, sofern vorhanden, nicht H ist;
X₁, X₂, X₃ und X₄ unabhängig voneinander aus der aus H, R, OH, -OR, -CO₂H, -CO₂R', F, Br, Cl, I, -CN, -SO₃H, -OSO₃H, NO₂, NH₂, -NHR und -NR₂ bestehenden Gruppe ausgewählt sind;
wobei R eine unverzweigte, verzweigte oder zyklische, gesättigte oder ungesättigte Alkylgruppe mit 1 bis 10 Kohlenstoffatomen ist, die unsubstituiert oder mit einem oder mehreren von OH, =O, SH, F, Br, Cl, I, NH₂, -NHR', -NR'₂, NO₂, -CO₂H, -CO₂R' und Epoxid substituiert ist;
und R' eine unverzweigte, verzweigte oder zyklische, gesättigte oder ungesättigte Alkylgruppe mit 1 bis 10 Kohlenstoffatomen ist, die unsubstituiert oder mit einem oder mehreren von OH, =O, SH, F, Br, Cl, I, NH₂, -NHR", -NR"₂, NO₂ und -CO₂H substituiert ist, wobei R" eine unverzweigte, verzweigte oder zyklische, gesättigte oder ungesättigte Alkylgruppe mit 1 bis 10 Kohlenstoffatomen ist;
mit der Maßgabe, dass die Verbindung nicht die gleiche Struktur wie Pelorol oder eine aus der aus Folgendem bestehenden Gruppe von Strukturen aufweist:

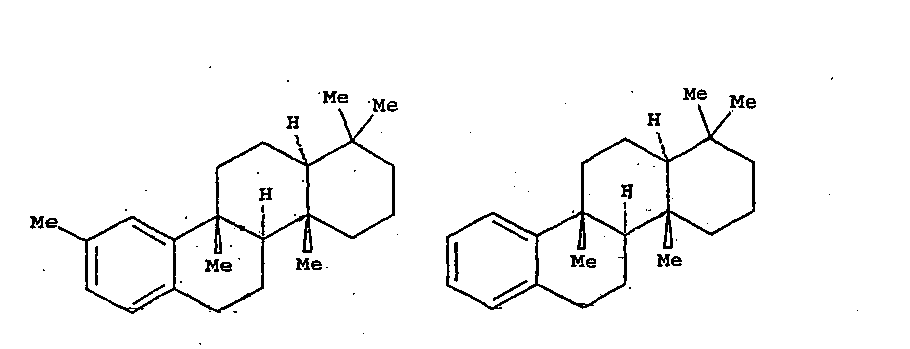

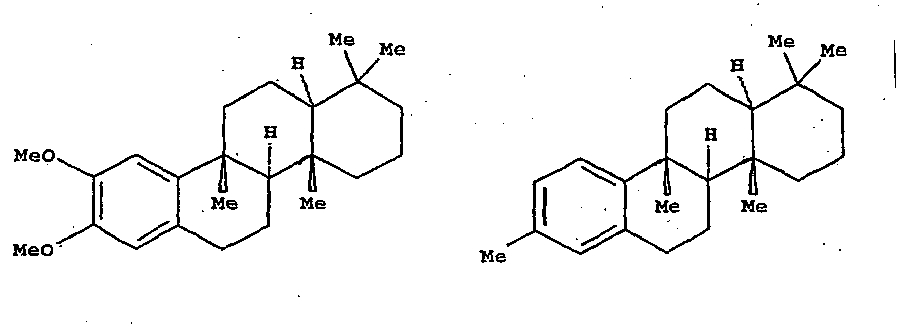

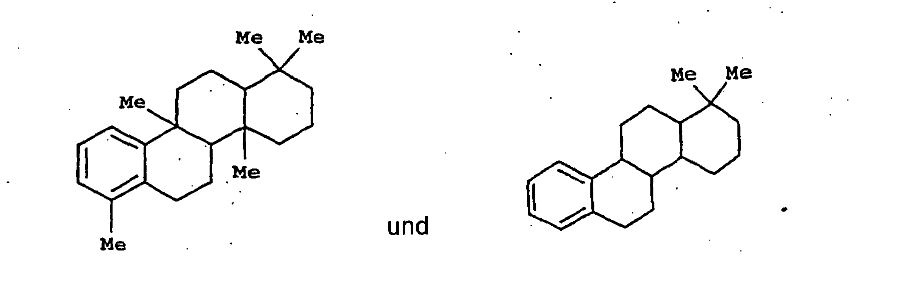

2. Verbindung nach Anspruch 1, worin Y₁ bis Y₆ unabhängig voneinander aus H, F, Br, Cl und I ausgewählt sind.

3. Verbindung nach Anspruch 1, worin Q -CH₂- ist.

4. Verbindung nach Anspruch 1, worin Q -CH₂CH₂- ist.

5. Verbindung nach Anspruch 1, worin Q -CH=CH-, -CH₂CH₂CH₂- oder -CH=CHCH₂- ist.

6. Verbindung nach einem der Ansprüche 1 bis 5, worin R₁ Methyl, Ethyl, -CH₂OH oder -CH₂OR' ist.

7. Verbindung nach einem der Ansprüche 1 bis 6, worin R₂ Methyl, Ethyl, -CH₂OH oder -CH₂OR' ist.

8. Verbindung nach einem der Ansprüche 1 bis 7, worin R' in R₁ auf Methyl, Ethyl, Propyl oder Butyl beschränkt ist.

9. Verbindung nach einem der Ansprüche 1 bis 8, worin R' in R₂ auf Methyl, Ethyl, Propyl oder Butyl beschränkt ist.

10. Verbindung nach Anspruch 8 oder 9, worin R' auf Methyl oder Ethyl beschränkt ist.

11. Verbindung nach einem der Ansprüche 1 bis 10, worin X₁ H, OH, R, OR, -CONH₂, -CONHR' oder -COR'₂ ist.

12. Verbindung nach einem der Ansprüche 1 bis 11, worin X₂ H, OH, R, OR, -CONH₂, -CONHR' oder -COR'₂ ist.

13. Verbindung nach einem der Ansprüche 1 bis 12, worin X₃ H, OH, R, OR, -CONH₂, -CONHR' oder COR'₂ ist.

14. Verbindung nach einem der Ansprüche 1 bis 13, worin R und R' in einem oder mehreren von X₁, X₂ und X₃ auf Methyl, Ethyl, Propyl und Butyl beschränkt sind.

15. Verbindung nach einem der Ansprüche 1 bis 10, worin X₁ H, OH oder -OCH₃ ist.

16. Verbindung nach einem der Ansprüche 1 bis 10 und 15, worin X₂ H, OH oder OCH₃ ist.

17. Verbindung nach einem der Ansprüche 1 bis 10 und 15, worin X₂ H, OCH₃ oder -NHOCH₃ ist.

18. Verbindung nach einem der Ansprüche 1 bis 10, 15, 16 und 17, worin X₃ H, OH oder OCH₃ ist.

19. Verbindung nach einem der Ansprüche 1 bis 18, worin X₄ H, R, OH, OR, CO₂H oder CO₂R' ist.

20. Verbindung nach einem der Ansprüche 1 bis 19, worin R und R' in X₄ auf Methyl, Ethyl, Propyl oder Butyl beschränkt sind.

21. Verbindung nach einem der Ansprüche 1 bis 18, worin X₄ H, R, OH, OCH₃, -CO₂H oder -CO₂CH₃ ist.

22. Verbindung nach Anspruch 1, die Homopelorol der Formel 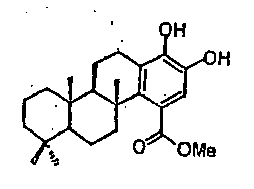 ist.

23. Verbindung nach Anspruch 1, die Dimethoxypelorol der Formel ist.

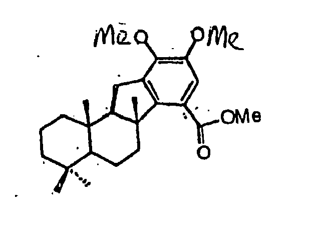

24. Verbindung nach Anspruch 1, die PNSR-4A der Formel ist.

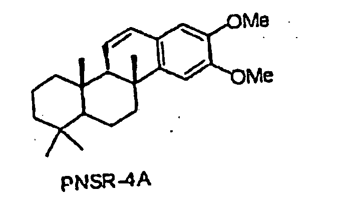

25. Verbindung nach Anspruch 1, die PNSR-15A der Formel ist.

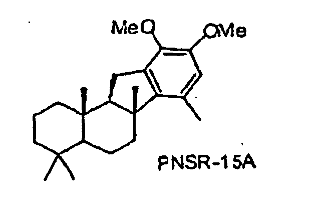

26. Verbindung nach Anspruch 1, die PNSR-16A der Formel ist.

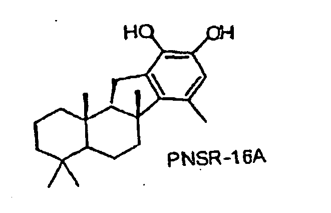

27. Verbindung nach Anspruch 1, die PNSR-17A der Formel 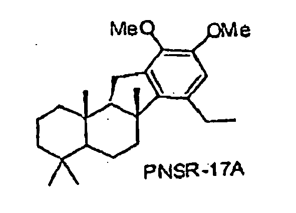 ist.

28. Verbindung nach Anspruch 1, die PNSR-18A der Formel ist.

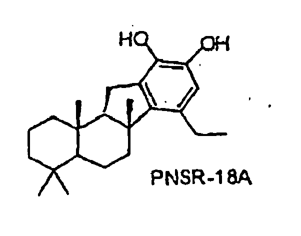

29. Verbindung nach einem der Ansprüche 1 bis 28 mit der Konfiguration S, R, R, S an C-5, C-8, C-9 bzw. C-10.

30. Verbindung nach einem der Ansprüche 1 bis 28 mit der Konfiguration R, S, S, R an C-5, C-8, C-9 bzw. C-10.

31. Verbindung nach einem der Ansprüche 1 bis 30 zur Verwendung als Modulator der SHIP-1-Aktivität.

32. Verbindung nach Anspruch 31, worin die Verbindung ein Agonist der SHIP-1-Aktivität ist.

33. Verbindung nach einem der Ansprüche 1 bis 32 zur Verwendung in einem Verfahren zur Behandlung oder Verhinderung einer bzw. eines Entzündungs-, Neoplasie-, Blutbildungs- oder Immunstörung oder -leidens.

34. Verfahren zur Herstellung einer Verbindung der Formel IA, worin R₁ bis R₄, X₁, X₃ und X₄ wie in Anspruch 1 definiert sind, 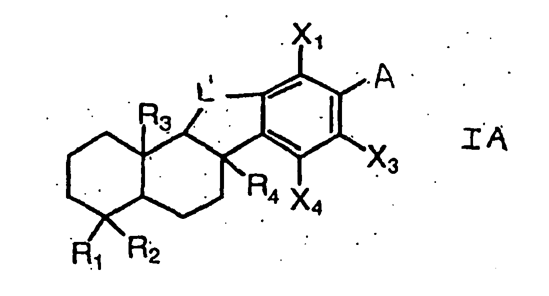 worin L' eine gesättigte oder ungesättigte Alkyl-Linkergruppe mit 1 bis 3 Kohlenstoffatomen ist; und A eine Aktivierungsgruppe ist; umfassend das Umsetzen einer Verbindung der Formel IIB: 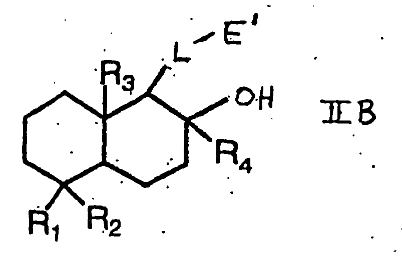 worin L fehlt oder eine gesättigte oder ungesättigte Alkyl-Linkergruppe mit 1 bis 3 Kohlenstoffatomen ist und E' eine elektrophile reaktive Gruppe ist; mit einer Verbindung der Formel III 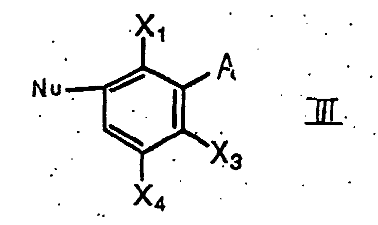 worin Nu eine Gruppe ist, welche die Verbindung der Formel III an Nu nucleophil macht, gefolgt von einer optionalen Reduktion und von Hydrolyse, um eine Verbindung der Formel IV 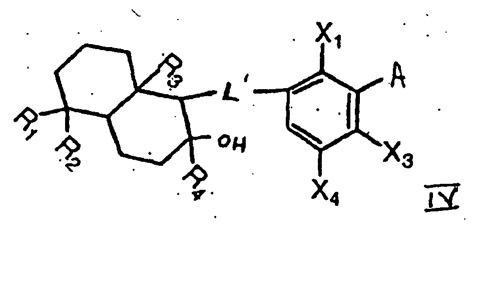 herzustellen, sowie Kondensieren der Verbindung der Formel IV, um eine Verbindung der Formel IA herzustellen.

35. Verfahren gemäß dem Reaktionsschema in Anspruch 34, worin die Verbindungen der Formel IIB folgende Struktur aufweisen: 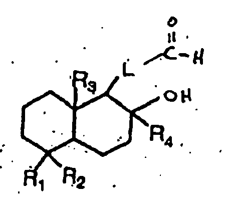 oder worin die Verbindungen der Formel IIB durch solche der Formel 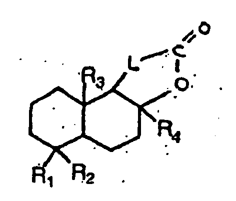 ersetzt sind.

36. Verfahren nach Anspruch 35, worin die Verbindungen der Formel IIB folgende Strukturen aufweisen:

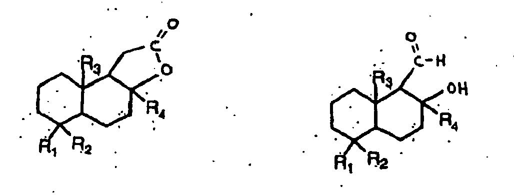

37. Verfahren nach einem der Ansprüche 35 bis 36, worin die Verbindung der Formel IIB Sclareolid ist oder von Sclareolid abgeleitet ist.

38. Verfahren nach einem der Ansprüche 34 bis 37, worin Nu Lithium ist.

39. Verfahren nach einem der Ansprüche 34 bis 39, worin A OCH₃ oder -NHOCH₃ ist.

40. Pharmazeutische Zusammensetzung, umfassend einen pharmazeutisch annehmbaren Träger und eine oder mehrere Verbindungen der Formel I oder pharmazeutisch annehmbare Salze davon, 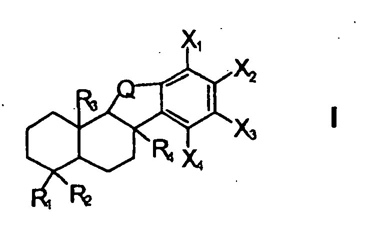 worin:
R₁ und R₂ unabhängig voneinander aus der aus -CH₃, -CH₂CH₃, -CH₂OH, -CH₂OR', -CHO, -CO₂H und -CO₂R' bestehenden Gruppe ausgewählt sind;
R₃ und R₄ unabhängig voneinander aus der aus H, -CH₃, -CH₂CH₃, -CH₂OH, -CH₂OR', -CHO, -CO₂H und -CO₂R' bestehenden Gruppe ausgewählt ist;
Q ein Kohlenstoffskelett ist, das aus der aus -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂- und -CY₁Y₂CY₃Y₄CY₅Y₆- bestehenden Gruppe ausgewählt ist; wobei Y₁, Y₂, Y₃, Y₄, Y₅ und Y₆ unabhängig voneinander aus der aus H, F, Br, Cl, I, OH, OR' und SH bestehenden Gruppe ausgewählt sind; oder eine beliebige der Gruppen Y₁/Y₂, Y₃/Y₄ und Y₅/Y₆ gegebenenfalls =O ist; oder Y₁/Y₃ gegebenenfalls ein Epoxid bildet; und zumindest eines von Y₁, Y₂, Y₃, Y₄, Y₅ und Y₆, sofern vorhanden, nicht H ist;
X₁, X₂, X₃ und X₄ unabhängig voneinander aus der aus H, R, OH, -OR, -CO₂H, -CO₂R', F, Br, Cl, I, -CN, -SO₃H, -OSO₃H, NO₂, NH₂, -NHR und -NR₂ bestehenden Gruppe ausgewählt sind;
wobei R eine unverzweigte, verzweigte oder zyklische, gesättigte oder ungesättigte Alkylgruppe mit 1 bis 10 Kohlenstoffatomen ist, die unsubstituiert oder mit einem oder mehreren von OH, =O, SH, F, Br, Cl, I, NH₂, -NHR', -NR'₂, NO₂, -CO₂H, -CO₂R' und Epoxid substituiert ist;
und R' eine unverzweigte, verzweigte oder zyklische, gesättigte oder ungesättigte Alkylgruppe mit 1 bis 10 Kohlenstoffatomen ist, die unsubstituiert oder mit einem oder mehreren von OH, =O, SH, F, Br, Cl, I, NH₂, -NHR", -NR"₂, NO₂ und -CO₂H substituiert ist, wobei R" eine unverzweigte, verzweigte oder zyklische, gesättigte oder ungesättigte Alkylgruppe mit 1 bis 10 Kohlenstoffatomen ist;
worin die eine oder die mehreren Verbindungen der Formel I nicht ausschließlich Pelorol sind.

41. Pharmazeutische Zusammensetzung nach Anspruch 40, die Pelorol umfasst.

42. Pharmazeutische Zusammensetzung nach Anspruch 40 oder 41, die eine Verbindung nach einem der Ansprüche 1 bis 32 umfasst.

43. Pharmazeutische Zusammensetzung nach einem der Ansprüche 40 bis 42 zur Verwendung in einem Verfahren zur Prävention oder Behandlung einer Immun-, Blutbildungs-, Entzündungs- oder Neoplasiestörung.

44. Pharmazeutische Zusammensetzung, umfassend einen pharmazeutisch annehmbaren Träger und eine oder mehrere Verbindungen der Formel IA oder pharmazeutisch annehmbare Salze davon zur Verwendung in einem Verfahren zur Prävention oder Behandlung einer Immun-, Blutbildungs-, Entzündungs- oder Neoplasiestörung, worin die Verbindung der Formel I folgende ist: 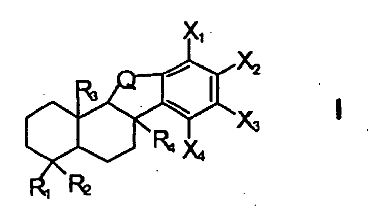 worin:
R₁ und R₂ unabhängig voneinander aus der aus -CH₃, -CH₂CH₃, -CH₂OH, -CH₂OR', -CHO, -CO₂H und -CO₂R' bestehenden Gruppe ausgewählt sind;
R₃ und R₄ unabhängig voneinander aus der aus H, -CH₃, -CH₂CH₃, -CH₂OH, -CH₂OR', -CHO, -CO₂H und -CO₂R' bestehenden Gruppe ausgewählt ist;
Q ein Kohlenstoffskelett ist, das aus der aus -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂- und -CY₁Y₂CY₃Y₄CY₅Y₆- bestehenden Gruppe ausgewählt ist; wobei Y₁, Y₂, Y₃, Y₄, Y₅ und Y₆ unabhängig voneinander aus der aus H, F, Br, Cl, I, OH, OR' und SH bestehenden Gruppe ausgewählt sind; oder eine beliebige der Gruppen Y₁/Y₂, Y₃/Y₄ und Y₅/Y₆ gegebenenfalls =O ist; oder Y₁/Y₃ gegebenenfalls ein Epoxid bildet; und zumindest eines von Y₁, Y₂, Y₃, Y₄, Y₅ und Y₆, sofern vorhanden, nicht H ist;
X₁, X₂, X₃ und X₄ unabhängig voneinander aus der aus H, R, OH, -OR, -CO₂H, -CO₂R', F, Br, Cl, I, -CN, -SO₃H, -OSO₃H, NO₂, NH₂, -NHR und -NR₂ bestehenden Gruppe ausgewählt sind;
wobei R eine unverzweigte, verzweigte oder zyklische, gesättigte oder ungesättigte Alkylgruppe mit 1 bis 10 Kohlenstoffatomen ist, die unsubstituiert oder mit einem oder mehreren von OH, =O, SH, F, Br, Cl, I, NH₂, -NHR', -NR'₂, NO₂, -CO₂H, -CO₂R' und Epoxid substituiert ist;
und R' eine unverzweigte, verzweigte oder zyklische, gesättigte oder ungesättigte Alkylgruppe mit 1 bis 10 Kohlenstoffatomen ist, die unsubstituiert oder mit einem oder mehreren von OH, =O, SH, F, Br, Cl, I, NH₂, -NHR", -NR"₂, NO₂ und -CO₂H substituiert ist, wobei R" eine unverzweigte, verzweigte oder zyklische, gesättigte oder ungesättigte Alkylgruppe mit 1 bis 10 Kohlenstoffatomen ist.

## Revendications

1. Composé de formule I ou sel de celui-ci, 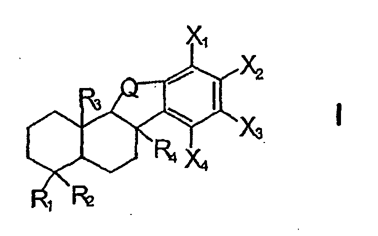 dans laquelle :
R₁ et R₂ sont indépendamment choisis dans le groupe consistant en : -CH₃, -CH₂CH₃, -CH₂OH, -CH₂OR', -CHO, -CO₂H et -CO₂R' ;
R₃ et R₄ sont indépendamment choisis dans le groupe consistant en : H, -CH₃, -CH₂CH₃, -CH₂OH, -CH₂OR', -CHO, -CO₂H et -CO₂R' ;
Q est un squelette carboné choisi dans le groupe consistant en : -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂- et -CY₁Y₂CY₃Y₄CY₅Y₆- ; où Y₁, Y₂, Y₃, Y₄, Y₅ et Y₆ sont indépendamment choisis dans le groupe consistant en : H, F, Br, Cl, I, OH, OR' et SH ; ou n'importe quel groupe de Y₁/Y₂, Y₃/Y₄ et Y₅/Y₆ peut être =O ; ou Y₁/Y₃ peuvent former un époxyde ; et, au moins l'un de Y₁, Y₂, Y₃, Y₄, Y₅ et Y₆ lorsqu'il est présent, n'est pas H ;
X₁, X₂, X₃ et X₄ sont indépendamment choisis dans le groupe consistant en : H, R, OH, -OR, -CO₂H, -CO₂R', F, Br, Cl, I, -CN, -SO₃H, -OSO₃H, NO₂, NH₂, -NHR et -NR₂ ; où R est un groupe alkyle comportant un à dix atomes de carbone saturé ou insaturé linéaire, ramifié ou cyclique qui est non substitué ou qui est substitué par un ou plusieurs parmi : OH, =O, SH, F, Br, Cl, I, NH₂, -NHR', -NR'₂, NO₂, -CO₂H, -CO₂R' et un époxyde ;
et R' est un groupe alkyle comportant un à dix atomes de carbone saturé ou insaturé, linéaire, ramifié ou cyclique qui est non substitué ou substitué par un ou plusieurs parmi : OH, =O, SH, F, Br, Cl, I, NH₂, -NHR'', -NR''₂, NO₂ et -CO₂H où R'' est un groupe alkyle comportant un à dix atomes de carbone saturé ou insaturé, linéaire, ramifié ou cyclique ;
à condition que le composé n'ait pas la structure précise du pélorol ou l'une quelconque du groupe de structures consistant en :

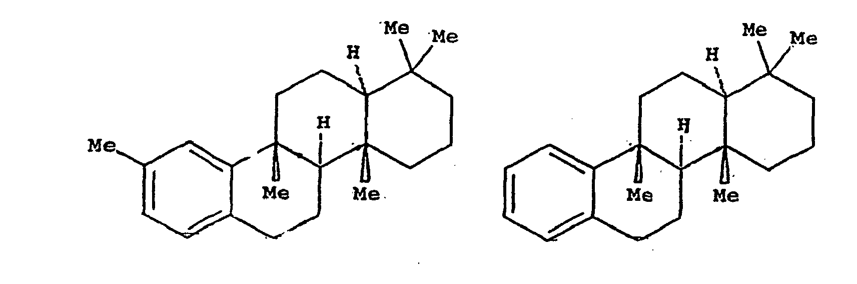

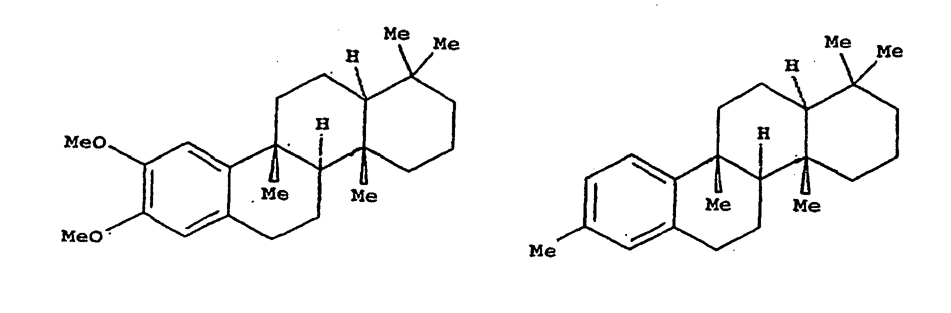

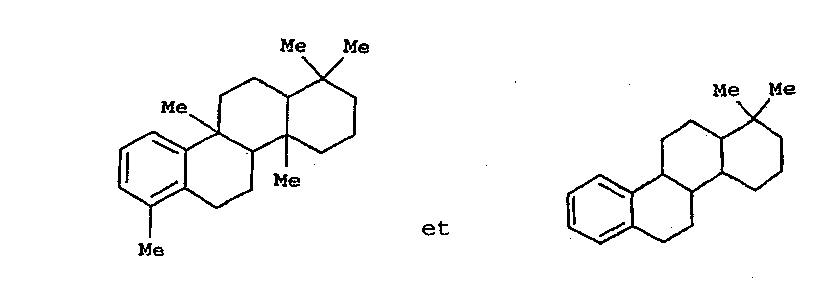

2. Composé selon la revendication 1, dans lequel Y₁ à Y₆ sont indépendamment choisis parmi H, F, Br, Cl et I.

3. Composé selon la revendication 1, dans lequel Q est -CH₂-.

4. Composé selon la revendication 1, dans lequel Q est -CH₂CH₂-.

5. Composé selon la revendication 1, dans lequel Q est -CH=CH-, -CH₂CH₂CH₂- ou -CH=CHCH₂-.

6. Composé selon l'une quelconque des revendications 1 à 5, dans lequel R₁ est un groupe méthyle, éthyle, -CH₂OH ou -CH₂OR'.

7. Composé selon l'une quelconque des revendications 1 à 6, dans lequel R₂ est un groupe méthyle, éthyle, -CH₂OH ou -CH₂OR'.

8. Composé selon l'une quelconque des revendications 1 à 7, dans lequel R' dans R₁ est limité à un groupe méthyle, éthyle, propyle ou butyle.

9. Composé selon l'une quelconque des revendications 1 à 8, dans lequel R' dans R₂ est limité à un groupe méthyle, éthyle, propyle ou butyle.

10. Composé selon la revendication 8 ou 9, dans lequel R' est limité à un groupe méthyle ou éthyle.

11. Composé selon l'une quelconque des revendications 1 à 10, dans lequel X₁ est H, OH, R, OR, -CONH₂, -CONHR' ou -COR'₂.

12. Composé selon l'une quelconque des revendications 1 à 11, dans lequel X₂ est H, OH, R, OR, -CONH₂, -CONHR' ou -COR'₂.

13. Composé selon l'une quelconque des revendications 1 à 12, dans lequel X₃ est H, OH, R, OR, -CONH₂, -CONHR' ou -COR'₂.

14. Composé selon l'une quelconque des revendications 1 à 13, dans lequel R et R' dans un ou plusieurs de X₁, X₂ et X₃ sont limités aux groupes méthyle, éthyle, propyle et butyle.

15. Composé selon l'une quelconque des revendications 1 à 10, dans lequel X₁ est H, OH ou -OCH₃.

16. Composé selon l'une quelconque des revendications 1 à 10 et 15, dans lequel X₂ est H, OH ou -OCH₃.

17. Composé selon l'une quelconque des revendications 1 à 10 et 15, dans lequel X₂ est H, OCH₃ ou -NHOCH₃.

18. Composé selon l'une quelconque des revendications 1 à 10, 15, 16 et 17, dans lequel X₃ est H, OH ou OCH₃.

19. Composé selon l'une quelconque des revendications 1 à 18, dans lequel X₄ est H, R, OH, OR, CO₂H ou CO₂R'.

20. Composé selon l'une quelconque des revendications 1 à 19, dans lequel R et R' dans X₄ sont limités à un groupe méthyle, éthyle, propyle ou butyle.

21. Composé selon l'une quelconque des revendications 1 à 18, dans lequel X₄ est H, R, OH, OCH₃, -CO₂H ou -CO₂CH₃.

22. Composé selon la revendication 1 qui est l'homopélorol de formule

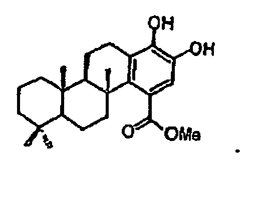

23. Composé selon la revendication 1 qui est le diméthoxypélorol de formule

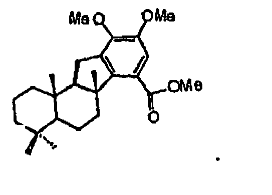

24. Composé selon la revendication 1 qui est le PNSR-4A de formule :

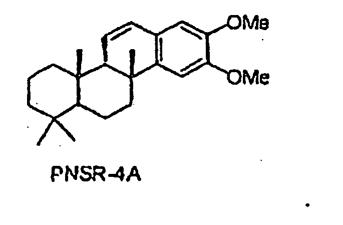

25. Composé selon la revendication 1 qui est le PNSR-15A de formule :

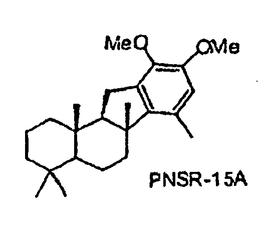

26. Composé selon la revendication 1 qui est le PNSR-16A de formule :

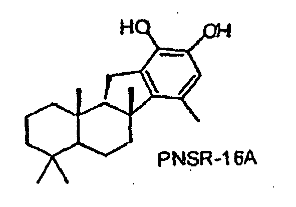

27. Composé selon la revendication 1 qui est le PNSR-17A de formule :

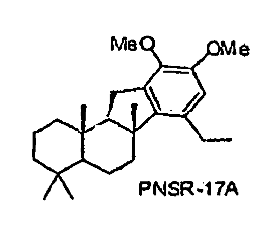

28. Composé selon la revendication 1 qui est le PNSR-18A de formule :

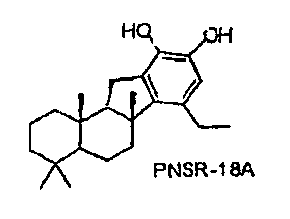

29. Composé selon l'une quelconque des revendications 1 à 28, ayant la configuration S, R, R, S en positions C-5, C-8, C-9 et C-10 respectivement.

30. Composé selon l'une quelconque des revendications 1 à 28, ayant la configuration R, S, S, R en positions C-5, C-8, C-9 et C-10 respectivement.

31. Composé selon l'une quelconque des revendications 1 à 30, pour une utilisation en tant que modulateur de l'activité de SHIP 1.

32. Composé selon la revendication 31, dans lequel le composé est un agoniste de l'activité de SHIP 1.

33. Composé selon l'une quelconque des revendications 1 à 32, pour une utilisation dans un procédé de traitement ou de prévention d'un trouble ou d'une infection inflammatoire, néoplasique, hématopoïétique ou immun.

34. Procédé de fabrication d'un composé de formule IA dans laquelle R₁ à R₄, X₁, X₃ et X₄ sont tels que définis dans la revendication 1, 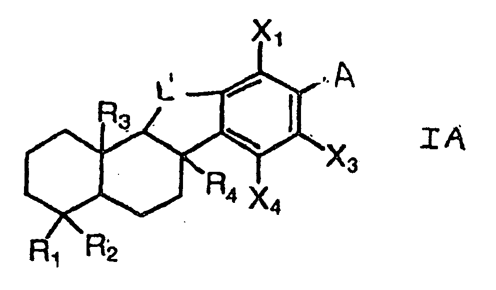 dans laquelle L' est un groupe de liaison de type alkyle en C1 à C3 saturé ou insaturé ; et A est un groupe d'activation ; comprenant la réaction d'un composé de formule IIB : 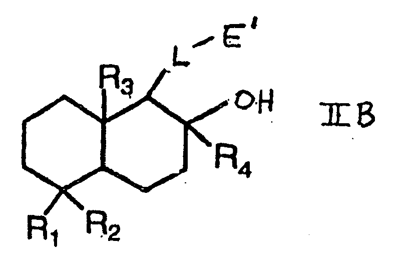 dans laquelle L est absent ou est un groupe de liaison de type alkyle en C1 à C3 saturé ou insaturé et E' est un groupe réactif électrophile ; avec un composé de formule III 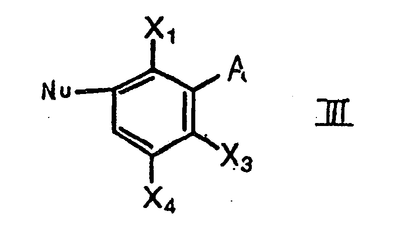 dans laquelle Nu est un groupe qui rend le composé de formule III nucléophile à Nu, suivi par une réduction facultative et par une hydrolyse, pour produire un composé de formule IV 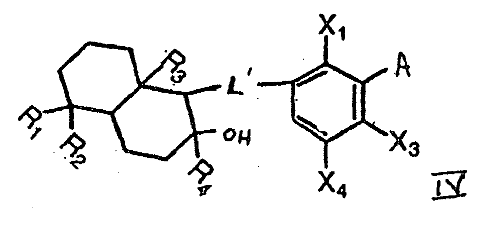 et une condensation du composé de formule IV pour produire un composé de formule IA.

35. Procédé employant le schéma de réaction selon la revendication 34 ; dans lequel les composés de formule IIB ont la structure : 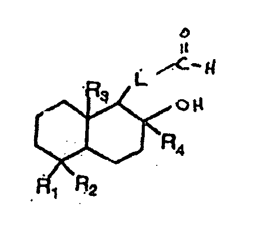 ou dans lequel les composés de formule IIB sont remplacés par ceux de formule :

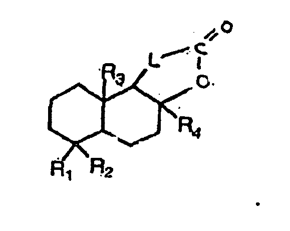

36. Procédé selon la revendication 35, dans lequel les composés de formule IIB ont les structures

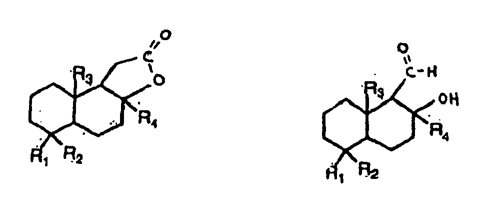

37. Procédé selon l'une quelconque des revendications 35 à 36, dans lequel le composé de formule IIB est le sclaréolide ou est dérivé du sclaréolide.

38. Procédé selon l'une quelconque des revendications 34 à 37, dans lequel Nu est le lithium.

39. Procédé selon l'une quelconque des revendications 34 à 39, dans lequel A est OCH₃ ou -NHOCH₃.

40. Composition pharmaceutique comprenant un véhicule pharmaceutiquement acceptable et un ou plusieurs composés de formule I ou des sels pharmaceutiquement acceptables de ceux-ci, 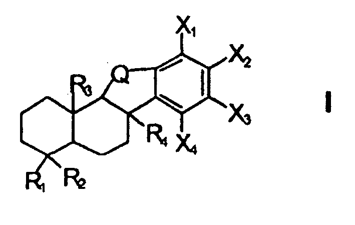 dans laquelle :
R₁ et R₂ sont indépendamment choisis dans le groupe consistant en : -CH₃, -CH₂CH₃, -CH₂OH, -CH₂OR', -CHO, -CO₂H et -CO₂R' ;
R₃ et R₄ sont indépendamment choisis dans le groupe consistant en : H, -CH₃, -CH₂CH₃, -CH₂OH, -CH₂OR', -CHO, - CO₂H et -CO₂R' ;
Q est un squelette carboné choisi dans le groupe consistant en : -CH₂-, -CY₁Y₂-, -CH₂CH₂-, -CH=CH-, - CY₁Y₂CY₃Y₄- , -CH₂CH₂CH₂-, -CH=CHCH₂-, -CH=CHCY₁Y₂- et - CY₁Y₂CY₃Y₄CY₅Y₆- ; où Y₁, Y₂, Y₃, Y₄, Y₅ et Y₆ sont indépendamment choisis dans le groupe consistant en : H, F, Br, Cl, I, OH, OR' et SH ; ou n'importe quel groupe de Y₁/Y₂, Y₃/Y₄ et Y₅/Y₆ peut être =O ; ou Y₁/Y₃ peuvent former un époxyde ; et, au moins l'un de Y₁, Y₂, Y₃, Y₄, Y₅ et Y₆ lorsqu'il est présent, n'est pas H ;
X₁, X₂, X₃ et X₄ sont indépendamment choisis dans le groupe consistant en : H, R, OH, -OR, -CO₂H, -CO₂R', F, Br, Cl, I, -CN, -SO₃H, -OSO₃H, NO₂, NH₂, -NHR et -NR₂ ;
où R est un groupe alkyle comportant un à dix atomes de carbone saturé ou insaturé linéaire, ramifié ou cyclique qui est non substitué ou qui est substitué par un ou plusieurs parmi : OH, =O, SH, F, Br, Cl, I, NH₂, -NHR', - NR'₂, NO₂, -CO₂H, -CO₂R' et un époxyde ;
et R' est un groupe alkyle comportant un à dix atomes de carbone saturé ou insaturé linéaire, ramifié ou cyclique qui est non substitué ou substitué par un ou plusieurs parmi : OH, =O, SH, F, Br, Cl, I, NH₂, -NHR'', -NR''₂, NO₂ et -CO₂H où R'' est un groupe alkyle comportant un à dix atomes de carbone saturé ou insaturé linéaire, ramifié ou cyclique ;
dans laquelle le ou les plusieurs composés de formule I ne sont pas uniquement le pélorol.

41. Composition pharmaceutique selon la revendication 40, comprenant le pélorol.

42. Composition pharmaceutique selon la revendication 40 ou 41, comprenant un composé selon l'une quelconque des revendications 1 à 32.

43. Composition pharmaceutique selon l'une quelconque des revendications 40 à 42 pour une utilisation dans un procédé de prophylaxie ou de traitement d'un trouble immun, hématopoïétique, inflammatoire ou néoplasique.

44. Composition pharmaceutique comprenant un véhicule pharmaceutiquement acceptable et un ou plusieurs composés de formule I ou sels pharmaceutiquement acceptables de ceux-ci pour une utilisation dans un procédé de prophylaxie ou de traitement d'un trouble immun, hématopoïétique, inflammatoire ou néoplasique, dans laquelle ledit composé de formule I est : 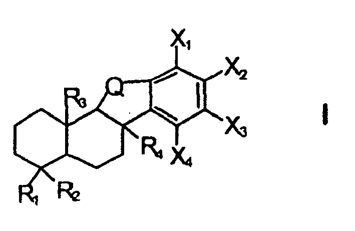 dans laquelle :
R₁ et R₂ sont indépendamment choisis dans le groupe consistant en : -CH₃, -CH₂CH₃, -CH₂OH, -CH₂OR', -CHO, -CO₂H et -CO₂R' ;
R₃ et R₄ sont indépendamment choisis dans le groupe consistant en : H, -CH₃, -CH₂CH₃, -CH₂OH, -CH₂OR', -CHO, - CO₂H et -CO₂R' ;
Q est un squelette carboné choisi dans le groupe consistant en : -CH₂-, -CY₁Y₂-, -CH₂CH₂-, -CH=CH-, -CY₁Y₂CY₃Y₄-, -CH₂CH₂CH₂-, -CH=CHCH₂-, -CH=CHCY₁Y₂- et -CY₁Y₂CY₃Y₄CY₅Y₆- ; où Y₁, Y₂, Y₃, Y₄, Y₅ et Y₆ sont indépendamment choisis dans le groupe consistant en : H, F, Br, Cl, I, OH, OR' et SH ; ou n'importe quel groupe de Y₁/Y₂, Y₃/Y₄ et Y₅/Y₆ peut être =O ; ou Y₁/Y₃ peuvent former un époxyde ; et, au moins l'un de Y₁, Y₂, Y₃, Y₄, Y₅ et Y₆ lorsqu'il est présent, n'est pas H ;
X₁, X₂, X₃ et X₄ sont indépendamment choisis dans le groupe consistant en : H, R, OH, -OR, -CO₂H, -CO₂R', F, Br, Cl, I, -CN, -SO₃H, -OSO₃H, NO₂, NH₂, -NHR et -NR₂ ;
où R est un groupe alkyle comportant un à dix atomes de carbone saturé ou insaturé linéaire, ramifié ou cyclique qui est non substitué ou qui est substitué par un ou plusieurs parmi : OH, =O, SH, F, Br, Cl, I, NH₂, -NHR', -NR'₂, NO₂, -CO₂H, -CO₂R' et un époxyde ;
et R' est un groupe alkyle comportant un à dix atomes de carbone saturé ou insaturé linéaire, ramifié ou cyclique qui est non substitué ou substitué par un ou plusieurs parmi : OH, =O, SH, F, Br, Cl, I, NH₂, -NHR'', -NR''₂, NO₂ et -CO₂H où R'' est un groupe alkyle comportant un à dix atomes de carbone saturé ou insaturé linéaire, ramifié ou cyclique.
